# EUROPEAN PATENT APPLICATION

(11) **EP 1 693 672 A1**
(43) Date of publication of application: **23.08.2006**
(21) Application number: 04819444.3
(22) Date of filing: 26.11.2004
(51) Int. Cl.: G01N 33/543

(54) **METHOD OF DETECTING SUBSTANCE TO BE ANALYZED**

(30) Priority: 28.11.2003 JP 2003398835; 22.03.2004 JP 2004082399
(71) Applicant: Olympus Corporation, Tokyo 151-0072 (JP)
(72) Inventor: SANUKI, Hiromi, Yokohama-shi, Kanagawa 227-0036 (JP); SAKAMOTO, Hiroko, Tokyo 141-0022 (JP); FUKUOKA, Morinao, Sagamihara-shi, Kanagawa 229-0003 (JP)
(74) Representative: von Hellfeld, Axel
(86) International application number: PCT/JP2004/017587
(87) International publication number: WO 2005/052583

(57) **Abstract**

An object of the invention is to provide a quick, easy, and highly-sensitive method of detecting a substance to be analyzed, and to provide a method of supporting the analysis results by quantitativeness even if the amount of samples to be handled is a very small amount. The invention is a method of detecting a substance to be analyzed, including: measuring a intensity of a signal derived from a labeled substance to be analyzed, in a time series during an increase in the signal intensity; and representing the signal intensity by a function of time, and using the quantitative value of the signal intensity for when the slope of an approximated line of the measured signal intensity is within a range between 0.5 and 1.5 times the slope of the approximated line of the previously measured signal intensity.

## Description

### BACKGROUND OF THE INVENTION

### Field of the Invention

The present invention relates to a method of detecting a substance to be analyzed such as a nucleic acid, highly-sensitively, easily, and quickly.

### Description of the Related Art

The development of techniques for diagnosing a disease by measuring a very small amount of substances in a living body, has been in progress. For example, there is a nucleic acid hybridization technique wherein the measurement is performed using a microarray having cDNA or an oligo fragment as a probe, in order to analyze the change in the mRNA expression level of a plurality of genes, or a gene mutation in a living tissue. Moreover, there has been developed a technique for measuring the weight of a proteinaceous molecule in a living tissue or a serum, using a protein array having an antibody or a peptide as a probe.

In order to establish the above methods as a technique for diagnosing a disease, it is required that the diagnosis can be easily, quickly, and accurately performed using a very small amount of an obtained sample. For example, it is ideal that a very small amount of mRNA or protein from several milligrams of tissues obtained by means of aspiration biopsy, a very small amount of cells in a body fluid, or several cells obtained by means of microdissection can be practically and easily measured. However, in the current state, a very small amount of a sample is amplified by several times of T7-aRNA amplifications of a very small amount of mRNA, and it is detected by nucleic acid hybridization. For this amplification step, it is necessary to prepare aRNA sample to be used for the hybridization. This preparation requires complicated operations over 2 to 4 days, and expensive reagents. Moreover, in an experimental system for handling a very small amount of sample, there is the difficulty of quantitatively handling the very small amount of sample itself. That is, in the case of handling a very small amount of sample, the fact is that the error rate with respect to the amount of sample is remarkably greater than that for the case of handling a larger amount of sample, and the existing problem is that the quantitativity of the measurement result is greatly affected by the amount of the handled sample. Furthermore, if the samples are quantified by measuring fluorescent substances or the like, the intensity of the fluorescence may differ between samples due to the difference (error or the like) in the sample amount, causing concern in that the previously determined condition for measuring the fluorescent substance may be an inappropriate condition for measuring the samples of that amount. Under such a condition, there is concern of the existence of samples whose intensity of the fluorescence has to be measured less than the measurement limit level, or saturation level or more, resulting in the occurrence of samples which can not be quantitatively measured. This decreases the advantage of the array system wherein a plurality of samples can be measured at the same time. This problem becomes more remarkable in a method of signal amplification by indirect labeling, or a method of signal amplification by using an enzyme, since the reaction velocity of the reaction of the signal amplification is greatly affected.

Incidentally, in nucleic acid hybridization, generally a single-strand of nucleic acid probe is hybridized with a labeled sample, and the resultant double-stand nucleic acid is detected. For detecting a protein, an antibody or a peptide is used as a probe. Conventionally, for such a detection of nucleic acid or protein, radioactive labeling and nonradioactive labeling have been used. Radioactive labeling of a sample is currently not mainstream method, since the available facility is limited and it can be hazardous although the sensitivity is excellent. As to the nonradioactive labeling, labeling with, for example a fluorescent substance, is general.

The method of labeling a nucleic acid itself is mainly classified into direct labeling and indirect labeling. As to the direct labeling method, a method of introducing a labeled nucleic acid into a sample at the time of its amplification is used in many cases. Among the nonradioactive labeling, for example, the direct labeling of a fluorescent substance is not sufficient in sensitivity, and furthermore background noise is relatively high, affecting the analysis of spots emitting weak signals in many cases. Therefore, a low expressive gene is difficult to analyze, requiring a large amount of sample used for one hybridization. Thus, as a sample, cDNA synthesized from a large amount of Total RNA is used or T7-aRNA amplified with over night incubating reaction is used actually. In order to solve this, indirect labeling has been developed.

In the developed method of using indirect labeling, the detection sensitivity is increased by conjugating a fluorescent substance to a substance which is specifically bindable to a substance conjugated to the sample (such as biotin-avidin system), or an antibody to the conjugated substance. Moreover, a method is also used in which an enzyme is conjugated to these specifically bindable substance or antibody, to activate the substrate of the enzyme (such as an HRP enzyme and a luminescent substrate ECL), so as to increase the detection sensitivity.

As an example of the indirect labeling method, a method described in Patent Document 1 (Japanese Patent Publication No. 2948904) is a method developed to amplify the detection signals system. However, this method uses an enzyme that is modified so as to specifically bind to the labeling of the substance to be detected. The substrate that has been modified to be detectable is activated by the enzyme, so that the activated substrate is deposited in any place where the receptor for the activated substrate is immobilized. Therefore, the detection signal of the modification substance of the deposited substrate is amplified, thereby increasing the detectivity or the quantitativeness of the substance to be analyzed in a specimen. As to an example of applying this method, there is reported a method of using a tyramide signal amplification kit commercially available from PerkinElmer, Inc., for detecting a microarray using cDNA probe on a slide glass (Non Patent Document 1; see below). The tyramide compound is radicalized by a catalytic action of horseradish peroxidase under the presence of hydrogen peroxide, so as to be covalently bonded to an aromatic amino acid in the vicinity thereof. The tyramide signal amplification kit utilizes this property, with an object of performing a highly-sensitive detection by applying a detectable modification substance (such as a fluorescent substance) to the tyramide compound.

However, if hybridization detection of a nucleic acid is performed by the abovementioned method using a slide glass array, all steps including washing after hybridization, blocking, conjugation reaction of the enzyme, washing, and deposition reaction of the substrate have to be performed in separate containers. Furthermore, several hours are required per one reaction. Consequently, the operation becomes complicated and time-consuming, in that it takes 2 to 3 days from hybridization to detection. Moreover, any aromatic amino acid serving as a receptor for the reporter (labeling) is not present on the slide glass array, requiring a step for previously loading the receptor to the array surface. This surface modification step is generally performed by coating using a proteinaceous substance (such as BSA and gelatin). Moreover, since the surface area is small in a planar slide glass array, the number of receptors that can be loaded is small, causing a problem in that, if a high expressive gene and a low expressive gene are measured at the same time, the receptor bonded by the tyramide compound is insufficient within the measurement range for relatively high expressions.

A carrier substance which immobilizes single-strand probes for nucleic acid hybridization includes: a backing with a large surface area capable of holding a liquid, which is made from such raw materials as a hollow fiber, a hollow fiber + gel, an aluminum oxide film, an etched silicon wafer, and a glass fiber; a filter made from nylon, nitrocellulose, or a resin; a gel matrix; or a three-dimensional porous carrier using metal oxide films described in Patent Document 2 (Japanese Patent Publication No. 3208390). The characteristic is such that they are capable of holding a liquid in itself, are capable of driving a liquid inside, and have a large surface area. Consequently, it is known that, the porous carrier has a larger amount of immobilized probes compared to that of a slide glass which is normally used for immobilizing probes, resulting in an improvement in the detection sensitivity and the detectable range.

However, in an experiment of nucleic acid hybridization in a case where the various porous carriers, such as, a metal oxide film, are used as a carrier for immobilizing the probes, there is a problem in that dust inside and outside of the sample solution is easily caught in holes of the carrier.
Patent Document 1: Japanese Patent Publication No. 2948904
Patent Document 2: Japanese Patent Publication No. 3208390
Non Patent Document 1: Karsten et. al., (Nucleic Acids Research), (England), 2002, vol. 30, No. 2 E4

Therefore, an object of the present invention is to provide a quick, easy, and highly-sensitive method of detecting a substance to be analyzed, and furthermore to provide a method of supporting the analysis results by quantitativeness even if the amount of samples to be handled is a very small amount.

### SUMMARY OF THE INVENTION

The present inventors have successfully found out that, in the method of detecting a substance to be analyzed, which is a method of depositing a substrate activated by an enzyme directly or indirectly bonded to the substance to be analyzed, onto a carrier, a substance to be analyzed such as a hybridized nucleic acid can be highly-sensitively, easily, and quickly detected by omitting a pretreatment step of loading an external receptor for the substrate, and thus they have come to the completion of the present invention. Moreover, they have found out that the difference in signal measurement results due to the difference in the amount of samples between very small amounts of samples can be effectively corrected, by quantifying an amount of a reference material (such as a gene and a protein) contained in the samples, and then based on the result, appropriately adjusting the measurement condition such as the time for measuring the respective samples and the reaction condition, and thus they have come to the completion of the present invention.

That is, a first aspect of the present invention is a method of detecting a substance to be analyzed, comprising:
(1) a step for measuring a intensity of a signal derived from a labeled substance to be analyzed, in a time series during an increase in the signal intensity;
(2) a step for comparing a specific signal intensity to a predetermined value; and
(3) a step for quantifying the substance to be analyzed, when the specific signal intensity comes to the predetermined value, using the signal intensity having that value.

Here, the labeling of the substance to be analyzed may be either direct labeling or indirect labeling.

A second aspect of the present invention is a method of detecting a substance to be analyzed, comprising the steps of:
measuring a intensity of a signal derived from a labeled substance to be analyzed, in a time series during an increase in the signal intensity; and
representing the signal intensity by a function of time, and
using the quantitative value of the signal intensity for when the slope of an approximated line of the measured signal intensity is within a range between 0.5 and 1.5 times the slope of the approximated line of a previously measured signal intensity.

A third aspect of the present invention is a method of detecting a substance to be analyzed according to the second aspect of the present invention, comprising, prior to the step of measuring the signal intensity:
(A) a step for supplying a sample solution containing the labeled substance to be analyzed, to a carrier having a immobilized probe for the substance to be analyzed; and
(B) a step for bonding the immobilized probe on the carrier, to the labeled substance to be analyzed.

A fourth aspect of the present invention is a method of detecting a substance to be analyzed according to the second aspect of the present invention, comprising, prior to the step of measuring the signal intensity:
a) a step for supplying a sample solution containing a conjugate A including one substance of a binding pair and the substance to be analyzed, to a carrier having a immobilized probe for the substance to be analyzed;
b) a step for reacting a conjugate B including an enzyme and a substance which is specifically bindable to said one substance of the binding pair, with the conjugate A that has been bonded to the immobilized probe on the carrier in the step a), so as to form a conjugate C comprising the conjugate A and the conjugate B; and
c) a step for reacting a conjugate D including a substrate for the enzyme in the conjugate B and a detectable labeling substance, with the conjugate C, so as to activate the substrate in the conjugate D, and bonding this activated conjugate D having the activated substrate, to the carrier without performing a pretreatment of attaching an external receptor for the substrate.

A fifth aspect of the present invention is a method of detecting a substance to be analyzed, comprising:
a') a step for supplying a sample solution containing a conjugate A including one substance of a binding pair and a substance to be analyzed, to a porous carrier having a immobilized probe for the substance to be analyzed; b') a step for reacting a conjugate B including an enzyme and a substance which is specifically bindable to said one substance of the binding pair, with the conjugate A that has been bonded to the immobilized probe on the carrier in the step a'), so as to form a conjugate C comprising the conjugate A and the conjugate B;
c') a step for reacting a conjugate D including a substrate for the enzyme in the conjugate B and a detectable labeling substance, with the conjugate C, so as to activate the substrate in the conjugate D, and bonding this activated conjugate D having the activated substrate, to the porous carrier without performing a pretreatment of attaching an external receptor for the substrate; and
d') a step for quantifying the labeling substance in the activated conjugate D on the porous carrier.

A sixth aspect of the present invention is a method of detecting a substance to be analyzed, comprising:
e') a step for supplying a sample solution containing a conjugate A including one substance of a binding pair and a substance to be analyzed, to a porous carrier having a immobilized probe for the substance to be analyzed, and then driving the sample solution to the inside and outside of this porous carrier;
f) a step for supplying a solution including a conjugate B including an enzyme and a substance which is specifically bindable to said one substance of the binding pair, to the porous carrier, then driving the solution to the inside and outside of this porous carrier, and reacting the conjugate B with the conjugate A that has been bonded to the immobilized probe on the carrier in the step e'), so as to form a conjugate C comprising the conjugate A and the conjugate B;
g') a step for supplying a solution containing a conjugate D including the substrate for the enzyme in the conjugate B and a detectable labeling substance, to the porous carrier, and then impregnating the solution into the porous carrier, reacting the conjugate D with the conjugate C, so as to activate the substrate in the conjugate D, and bonding the activated conjugate D to the porous carrier; and
h') a step for quantifying the labeling substance in the conjugate D on the porous carrier.

In this sixth aspect of the present invention, the activated substrate may be bonded to the external receptor by performing a pretreatment of loading an external receptor for the substrate, or the activated substrate may be directly bonded to the carrier without performing the pretreatment of loading the external receptor.

A seventh aspect of the present invention is a method of detecting a substance to be analyzed, according to any one of the first aspect through the sixth aspect of the present invention, wherein the steps (1) to (3); (A) to (B); a) to c); a') to c'); or e') to g') are performed within a range between 20°C to 70°C.

An eighth aspect of the present invention is a method of detecting a substance to be analyzed, according to any one of the first aspect through the sixth aspect of the present invention, wherein the steps (1) to (3); (A) to (B); a) to c); a') to c'); or e') to g') are performed within a range between 20°C and 70°C, at approximately the same temperature.

A ninth aspect of the present invention is a method of detecting a substance to be analyzed, according to any one of the third aspect through the sixth aspect of the present invention, comprising a step for washing the carrier: after the step (B) but before the signal intensity measurement; after the step c) but before the signal intensity measurement; after the step c') but before the step d'); or after the step g') but before the step h').

A tenth aspect of the present invention is a method of detecting a substance to be analyzed, according to the fifth aspect of the present invention, wherein the step d') is performed a plurality of times at optional time intervals after the step c').

An eleventh aspect of the present invention is a method of detecting a substance to be analyzed, according to the sixth aspect of the present invention, wherein the step h') is performed a plurality of times at optional time intervals after the step g').

A twelfth aspect of the present invention is a method of detecting a substance to be analyzed, according to either the tenth aspect or the eleventh aspect of the present invention, wherein: in the step d') or h'), the measured signal intensity is represented by a function of time, and the quantification is performed using the signal intensity for when the slope of an approximated line of the measured signal intensity is within a range between 0.5 and 1.5 times the slope of the approximated line of the previously measured signal intensity.

A thirteenth aspect of the present invention is a method of detecting a substance to be analyzed, comprising the following steps a') to c') or e') to g'):
a') a step for supplying a sample solution containing a conjugate A including one substance of a binding pair and a substance to be analyzed, to a porous carrier having a immobilized probe for the substance to be analyzed;
b') a step for reacting a conjugate B including an enzyme and a substance which is specifically bindable to said one substance of the binding pair, with the conjugate A that has been bonded to the immobilized probe on the carrier in the step a'), so as to form a conjugate C comprising the conjugate A and the conjugate B;
c') a step for reacting a conjugate D including a substrate for the enzyme in the conjugate B and a detectable labeling substance, with the conjugate C, so as to activate the substrate in the conjugate D, and bonding this activated conjugate D having the activated substrate, to the porous carrier without performing a pretreatment of attaching an external receptor for the substrate;
e') a step for supplying a sample solution containing a conjugate A including one substance of a binding pair and the substance to be analyzed, to a porous carrier having a immobilized probe for the substance to be analyzed, and then driving the sample solution to the inside and outside of this porous carrier;
f) a step for supplying a solution containing a conjugate B including an enzyme and a substance which is specifically bindable to said one substance of the binding pair, to the porous carrier, then driving the solution to the inside and outside of this porous carrier, and reacting the conjugate B with the conjugate A that has been bonded to the immobilized probe on the carrier in the step e'), so as to form a conjugate C comprising the conjugate A and the conjugate B; and
g') a step for supplying a solution containing a conjugate D including the substrate for the enzyme in the conjugate B and a detectable labeling substance, to the porous carrier, and then impregnating the solution into the porous carrier, reacting the conjugate D with the conjugate C, so as to activate the substrate in the conjugate D, and bonding the activated conjugate D to the porous carrier; and
further comprising, after the step c') or g'):
   x-1) a step for measuring only a signal intensity from the activated conjugate D in a specific spot on the porous carrier, to confirm in time series whether or not the signal intensity from the specific spot comes to the predetermined signal intensity; and
   x-2) a step for, right after confirming that it comes to the predetermined signal intensity in the step x-1), stopping reactions in other spots on the porous carrier, starting to measure the signal intensity from the other spots, representing the signal intensity by a function of time, and measuring the amount using the signal intensity for when the slope of an approximated line of the measured signal intensity is within a range between 0.5 and 1.5 times the slope of the approximated line of the previously measured signal intensity.

A fourteenth aspect of the present invention is a method of detecting a substance to be analyzed, according to the thirteenth aspect of the present invention, wherein the specific spot in the step x-1) is a spot having a immobilized probe for a reference material.

A fifteenth aspect of the present invention is a method of detecting a substance to be analyzed, according to the fourteenth aspect of the present invention, wherein there are a plurality of the specific spots, and probes whose immobilized amounts are adjusted in a dilution series at a constant ratio are immobilized in the respective spots.

A sixteenth aspect of the present invention is a method of detecting a substance to be analyzed, according to the ninth aspect of the present invention, wherein the amount of washing liquid used in the washing step is greater than the amount of solution used in the step (B), c), c'), or g').

A seventeenth aspect of the present invention is a method of detecting a substance to be analyzed, according to any one of the first aspect, the second aspect, the fifth aspect, the sixth aspect, and the thirteenth aspect of the present invention, wherein the substance to be analyzed is a nucleic acid, a sugar, a protein, a peptide, or a modified substance thereof.

An eighteenth aspect of the present invention is a method of detecting a substance to be analyzed, according to the seventeenth aspect of the present invention, wherein, if the substance to be analyzed is a nucleic acid, the amount of the substance to be analyzed is within a range between 0.01 ng and 1.0 µg.

A nineteenth aspect of the present invention is a method of detecting a substance to be analyzed, according to any one of the fourth aspect, the fifth aspect, the sixth aspect, and the thirteenth aspect of the present invention, wherein the enzyme is at least one type of enzyme selected from a group consisting of an oxidoreductase, a hydrolase, a lyase, a transferase, an isomerase, and a ligase.

A twentieth aspect of the present invention is a method of detecting a substance to be analyzed, according to the nineteenth aspect of the present invention, wherein the amount of the enzyme is within a range between 0.8 pmol/ml and 6 pmol/ml.

A twenty-first aspect of the present invention is a method of detecting a substance to be analyzed, according to any one of the fourth aspect, the fifth aspect, the sixth aspect, and the thirteenth aspect of the present invention, wherein the binding pair is selected from a group consisting of biotin-avidin, biotin-streptavidin, antigen-antibody, and ligand-receptor.

A twenty-second aspect of the present invention is a method of detecting a substance to be analyzed, according to any one of the fourth aspect, the fifth aspect, the sixth aspect, and the thirteenth aspect of the present invention, wherein the substrate is a substituted phenol or a phosphorylated substituted phenol.

A twenty-third aspect of the present invention is a method of detecting a substance to be analyzed, according to any one of the fourth aspect, the fifth aspect, the sixth aspect, and the thirteenth aspect of the present invention, wherein the enzyme is a peroxidase, and the step c), c'), or g') is performed under the presence of hydrogen peroxide having a concentration of 0.00008 to 0.0003%.

A twenty-fourth aspect of the present invention is a method of detecting a substance to be analyzed, according to any one of the first aspect, the second aspect, the fifth aspect, the sixth aspect, and the thirteenth aspect of the present invention, wherein the sample solution includes a surfactant.

A twenty-fifth aspect of the present invention is a method of detecting a substance to be analyzed, according to the ninth aspect of the present invention, wherein the washing solution used in the washing step includes a surfactant.

A twenty-sixth aspect of the present invention is a method of detecting a substance to be analyzed, according to any one of the fifth aspect, the sixth aspect, and the thirteenth aspect of the present invention, wherein the material of the porous carrier is a metal oxide film.

A twenty-seventh aspect of the present invention is a method of detecting a substance to be analyzed, according to any one of the fifth aspect, the sixth aspect, and the thirteenth aspect of the present invention, wherein the porous carrier is pretreated with a compound containing an aromatic amino acid in at least a part thereof.

A twenty-eighth aspect of the present invention is a method of detecting a substance to be analyzed, according to any one of the first aspect, the second aspect, the fifth aspect, the sixth aspect, and the thirteenth aspect of the present invention, wherein there are one or more types of the substances to be analyzed, and one or more types of probes for the respective substances to be analyzed are immobilized in respectively separate spots on a porous carrier in a single reaction container.

A twenty-ninth aspect of the present invention is a method of detecting a substance to be analyzed, according to either the tenth aspect or the eleventh aspect of the present invention, wherein there are two or more of the substances to be analyzed, the method further comprising a step for comparing the quantitative values of the two or more substances to be analyzed, using the signal intensity for when the signal intensity from the labeling substance is a predetermined value.

A thirtieth aspect of the present invention is a method of detecting a substance to be analyzed, according to any one of the first aspect, the second aspect, the fifth aspect, the sixth aspect, and the thirteenth aspect of the present invention, wherein: the diameter or the diagonal length of the spots if the probe immobilizable area in the spots is two-dimensional, or the diameter or the diagonal length of the cross-section of the spots in parallel with the base portion of the carrier if the probe immobilizable area in the spots is three-dimensional, is within a range between 50 and 500 µm; and the number of the spots on a single carrier is 20 or more.

A thirty-first aspect of the present invention is a reagent kit for detecting a substance to be analyzed having in respectively separate containers:
a blocking agent containing a buffer solution containing a monovalent or divalent cation, and at least one type selected from a group consisting of BSA, gelatin, and skim milk;
an enzyme preparation containing a buffer solution containing a monovalent or divalent cation, and at least one type of enzyme selected from a group consisting of oxidoreductase, hydrolase, lyase, transferase, isomerase, and ligase;
a substrate-containing agent containing a conjugate D including a detectable labeling substance and a substrate for the enzyme in the enzyme preparation; and
a washing agent containing a nonionic surfactant and a buffer solution containing a monovalent or divalent cation.

A thirty-second aspect of the present invention is a reagent kit for detecting a substance to be analyzed having in respectively separate containers:
a blocking agent containing 0.1M to 1M NaCl, 3mM to 60mM sodium phosphate, and 0.1% to 10% of at least one type selected from a group consisting of BSA, gelatin, and skim milk;
an enzyme preparation containing 0.8 pmol/ml to 6 pmol/ml peroxidase, and a buffer solution containing a monovalent or divalent cation;
a substrate-containing agent containing a conjugate D including a detectable labeling substance and a substrate for the enzyme in the enzyme preparation;
a substrate dissolving solution containing 0.00008% to 0.0003% hydrogen peroxide; and
a washing liquid containing 0.1 % to 1% nonionic surfactant, 0.1 M to 1M NaCI, and 3mM to 60mM sodium phosphate.

By using the method of the present invention, the signal intensity of the detection spot is greatly increased, and it becomes possible to detect a substance to be analyzed that has been undetectable in a conventional method, such as a low expressive gene group. Furthermore, from a low expressive biological substance to a high expressive biological substance, detection can be performed highly-accurately and quantitatively, regardless of the high or low expression level.

Moreover, the improvement of the detection sensitivity enables accurate detection of a signal even if the amount of sample such as a nucleic acid used for hybridization is very small. Furthermore, if the amount of the obtainable sample is extremely small, for example if a substance to be analyzed such as a very small amount of cells sampled from a clinical specimen by means of microdissection, or a tissue sampled from a living body by means of biopsy, in particular a very small amount of nucleic acid or protein from a tissue sampled by means of fine needle aspiration biopsy (for example, 0.1 ng or more in the case of a nucleic acid such as RNA and DNA), is handled, then quantitative detection becomes possible, and it can be expected that the application range of a microarray is broadened from clinical research to diagnosis.

In a conventional method, dust on the array surface or unevenness of the array surface might become visible in the analysis image in some cases, requiring a correction of removing the dust on the image or a correction of the background value at the time of signal analysis. However, in the method of the present invention, in particular, by using the method of driving the solution to the inside and outside of the carrier, then after the sample solution is supplied to the porous carrier, the exposure time up to detection can be greatly shortened. Therefore, the dust or the unevenness of the array surface does not affect the image, and the time required for the image analysis can be shortened.

Furthermore, since the background value is almost even, the reliability of the analysis data of the signal spot is improved. Therefore, it becomes unnecessary to perform a filtering treatment for removing dust at the time of preparing the sample, or a filtering treatment of each solution to be put on the chip. Moreover, up to now, arrays having dust or unevenness more than a fixed level, have been removed during the array production. However, since these arrays can be used for analysis by the present invention, there is obtained an additional effect of improving the yield of array production and improving the production efficiency.

Using the method of the present invention, complicated operations over a long time that have been required in a glass array of a conventional method, can be performed by simple operations where all steps can be performed in the same chamber. Moreover, if the solution is driven to the inside and outside of the carrier, the reaction proceeds for an even shorter time, so that all steps can be processed in an even shorter time.

In the method of the present invention, by performing the each step in a same chamber whose temperature is controlled, the reaction strength due to the temperature can be controlled in each experiment, enabling to obtain reproducible data. Moreover, by performing the each step in the same chamber, the effect of temperature change occurring during the transition from step to step can be minimized. Furthermore, complicated operations such as transferring to another chamber become unnecessary, and an easy and quick experiment can be performed.

In the method of the present invention, in the case where the signal intensity of a specific spot on a porous carrier is monitored, and when the specific signal intensity comes to a predetermined value, reactions in other spots are stopped to measure the signal intensity, then the reaction time between the conjugate C and the conjugate D in the pertinent other spots, and the reaction time between the porous carrier and the activated substrate in the conjugate D are indirectly controlled. Therefore, even if the amount of the substance to be analyzed can not be accurately measured, measurement within a range of the detection limit level or more but less than the saturation level can be performed in many of the pertinent other spots. That is, the degree of the signal intensity of a specific spot (immobilized probe for an internal reference material, or the like) that enables the measurement in many of the pertinent other spots within the detection range, can be set by a preliminary experiment regarding the degree of sample amount and the reaction time, considering the ratio of quantity between the substance to be analyzed and the internal reference material.

Consequently, according to this method, even if the amount of sample to be analyzed is not accurate, the signal intensity of a specific spot is monitored, and when the signal intensity comes to a predetermined value, reactions in other spots are stopped, to measure the signal intensitys in the pertinent other spots when the ratio of change in the signal intensity is within a predetermined range, so that it becomes possible to keep the quantitativeness in the measurement result between the respective spots.

By using the reagent kit for detecting a substance to be analyzed of the present invention, it becomes possible to easily perform highly-sensitive detection.
1. In the method of detecting a substance to be analyzed of the present invention, the intensity of a signal derived from a labeled substance to be analyzed, is measured in time series, and (i), the signal intensity of a spot is compared with a predetermined value, and the signal intensity having a value of when it comes to that value, is adopted, or (ii) the signal intensity is represented by a function of time, and the quantitative value of the signal intensity for when the slope of an approximated line of the measured signal intensity is within a range between 0.5 and 1.5 times the slope of the approximated line of the previously measured signal intensity, is adopted. Therefore, it is possible to confirm whether or not the signal intensity is an appropriate value for measuring with quantitativeness, or whether or not the measurement time and measurement signal intensity are in a proportional relation (that is, whether or not the signal intensity is increased in an approximately linear manner), and it is possible to use the measurement value under the optimum measurement condition for quantitation. Consequently, it becomes possible to perform quantitation more accurately, without using data for the case where the signal intensity is extremely small, or the case where the signal intensity is saturated in the measurement system.
2. In the present invention, a detectably labeled substrate is activated by the enzyme in the conjugate (conjugate C) including the binding pair and the substance to be analyzed, and reacted with the carrier surface in the vicinity of a probe capturing the substance to be analyzed (hybridizing if the substance to be analyzed is a nucleic acid). The detectably labeled substrate is reacted with, in particular a receptor (external receptor) loaded to the carrier surface, or an OH group on the surface of the substrate, causing a chemical bond (deposition reaction). Consequently, by measuring the detectable label bonded with the substrate, the amount of the captured substance to be analyzed can be measured. In this manner, since the label that is chemically bonded with the carrier is detected, even if a washing step for washing the carrier is introduced before the step for measuring the signal intensity, the label is not easily peeled off but unreacted substances can be easily removed. Consequently, causative substances of noise can be removed without damaging the substance emitting a signal from the carrier. Consequently, signal-noise ratio is increased. Moreover, in the washing step, by using the washing liquid having an amount more than the capacity of solutions used in the respective previous steps, and by passing the washing liquid back and forth through the carrier, unreacted substances can be more quickly and cleanly washed from the carrier. Furthermore, since the level of the washing liquid comes to a position higher than the level of the reaction liquid on the side face of the respective spots for performing various reactions, residue on the spot side face which often affects the background value, is not left after washing, and data having a lower background value can be obtained. The chemical bond of the substrate is called deposition. By using a signal amplification method by means of deposition, the amount of the detectable labeling substance per substance to be analyzed captured by the carrier is greatly increased compared to the case where the substance to be analyzed is detectably labeled directly. Therefore even a low expressive gene having a small amount for hybridization becomes detectable, since the amount of the detectable labeling substance per the substance to be analyzed is increased.
3. In the present invention, as a carrier substantially capable of holding liquid in the carrier for immobilizing the probe, a three-dimensional porous carrier can be used. For example, if an aluminum anode oxide film is used for the carrier, the surface area becomes about 500 times larger compared to a planar glass array. Consequently, since not only the probes but also the deposition substances used for the detection system can be adhered more, the rate of strengthening the signal is greater compared to the conventional case of using a glass array. Moreover, since the three-dimensional porous carrier has depth, if the detection is performed from the top, the vertical signal intensity is superposed, and thus the effect also acts to strengthen the signal.
4. In the present invention, if the carrier for immobilizing the probe is porous, a liquid can be passed therethrough. Therefore, the reaction for bonding the conjugate B to the conjugate A that has been bonded to the immobilized probe, so as to form the conjugate C can be performed while the solution containing the conjugate B comprising an enzyme and the substance which is specifically bindable to the one substance of the binding pair (the other substance of the binding pair), is passed back and forth therethrough. Therefore, the amount of liquid used in the each reaction may be small. Moreover, the probe and the substance to be analyzed, and the conjugate B and the conjugate A bonded to the immobilized probe, come into contact more often, so that the reaction can be easily and quickly performed.
5. In the present invention, a substrate is impregnated into a carrier substantially capable of holding liquid, which is then left to stand, so as to perform a reaction between the conjugate A and the conjugate B, a reaction between the conjugate C and the conjugate D, and a reaction between the carrier surface and the substrate activated by the enzyme. Then, a large number of nucleic acid probes are immobilized on the carrier, to which a large number of substances to be analyzed are hybridized, increasing the frequency of contact between the enzyme in the conjugate D and the substrate. Consequently, the deposition reaction of the substrate with respect to the carrier can be generated concentratingly in the vicinity of the nucleic acid probe bonded with the substance to be analyzed, thus increasing the detection sensitivity of the method of the present invention.
6. In the present invention, if the detection is performed with a CCD camera, it is possible to obtain a sufficient signal even if the exposure time is short. Therefore, noise of dust in the sample, and of dust and unevenness of the carrier can be greatly reduced. Consequently, it becomes unnecessary to remove dust in the sample or reagents at the time of preparing the sample. Moreover, carriers having unevenness or dust can be used.
7. In the present invention, in the step for measuring the labeling substance, it is confirmed in time series whether or not the signal intensity from the activated conjugate D in the specific spot on the porous carrier comes to a predetermined signal intensity. Right after confirming that it comes to the that signal intensity, the signal intensity from the activated conjugate D in the other spots is quantified. In this case, if the probe for the reference material is immobilized on the specific spot, it can be expected that the substance to be analyzed existing at a constant ratio with the reference material can be measured within a range of the measurement limit level or more but less than the saturation level.

### BRIEF DESCRIPTION OF THE DRAWINGS

FIG 1 shows the number of dust particles in the analysis image in the present invention.
FIG 2 shows the number of analyzable spots in the present invention.
FIG 3 shows the number of analyzable spots when a very small amount of sample is used, in the present invention.
FIG 4 shows the number of analyzable spots depending on the presence/absence of an external receptor, in the present invention.
FIG 5 shows the difference in the number of analyzable spots between the method of the present invention and a conventional method.
FIG 6 shows the change in the detection amount of a low expressive gene and a high expressive gene, by changing the time for the deposition of the activated substrate onto the carrier, in the method of the present invention.
FIG 7 shows the relation between the concentration of the substance to be analyzed which is to be used, and the obtained signal intensity, in the method of the present invention.
FIG 8 shows the correlation between the expression level ratio by a conventional direct labeling method, and the expression level ratio by the signal amplification method of the present invention.
FIG 9 shows the results of examination of inhibition of cdc2 kinase activity by Olomoucine, using the method of the present invention.

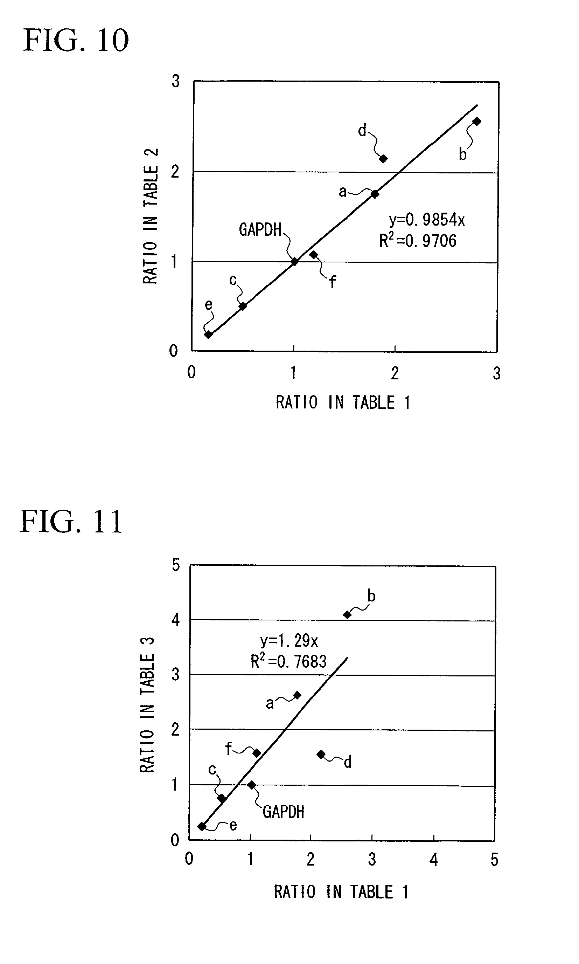

FIG 10 shows the correlation between the expression level ratio measured without matching the amount of the substance to be analyzed, and the expression level ratio measured with matching the amount of the substance to be analyzed, using the method of the present invention.
FIG 11 shows the correlation between the expression level ratio measured without matching the amount of the substance to be analyzed using the method of the present invention, and the expression level ratio measured without matching the amount of the substance to be analyzed using a conventional method.

### DETAILED DESCRIPTION OF THE PREFEERED EMBODIMENTS

The method of detecting a substance to be analyzed of the present invention comprises:
(1) a step for measuring a intensity of a signal derived from a labeled substance to be analyzed, in a time series during an increase in the signal intensity;
(2) a step for comparing a specific signal intensity to a predetermined value; and
(3) a step for quantifying the substance to be analyzed, when the specific signal intensity comes to the predetermined value, using the signal intensity having that value.

Here, the "signal" to be measured (quantified) has a spectroscopic property, specifically it may be any one of fluorescence, absorption, and chromophore.

Moreover, "during an increase in the signal intensity" includes a case where a reaction between the detectably labeled substance to be analyzed and the probe which is specifically bindable to the substance to be analyzed, is in progress (a case where the substance to be analyzed is directly labeled).

Furthermore, it includes the case where, after the bonding of the substance to be analyzed and the conjugate (the conjugate B below) which includes an enzyme and specifically binds to the substance bonded with the substance to be analyzed, then the signal intensity derived from the converted substrate due to a conversion reaction of the substrate catalyzed by the enzyme, is increased (the case where the substance to be analyzed is indirectly labeled).

In this manner, during an increase in the signal intensity, the specific signal intensity is compared to the predetermined value, and when it comes to the predetermined value, the quantification is performed using the signal intensity Here, the "predetermined value" is a value within a range of the detection limit or more but less than a value with which it is considered that the signal intensity is approximately saturated in the detection system used. Preferably, it is, in a function of time change in the signal intensity, a value (signal intensity) of when the slope of the approximated line at the time of measurement is approximately constant. More preferably, it is a value of the signal intensity for when the slope of an approximated line of the measured signal intensity is within a range between 0.5 and 1.5 times the slope of the approximated line of the previously measured signal intensity

Consequently, the method of detecting a substance to be analyzed of the present invention, is characterized in that in a method of detecting a substance to be analyzed, a intensity of a signal derived from the labeled substance to be analyzed, is measured in time series during an increase in the signal intensity, and the signal intensity is represented by a function of time, and the quantitative value of the signal intensity for when the slope of an approximated line of the measured signal intensity is within a range between 0.5 and 1.5 times the slope of the approximated line of the previously measured signal intensity, is used.

The method of detecting a substance to be analyzed of the present invention, comprises, prior to measuring the signal intensity:
(A) a step for supplying a sample solution containing the labeled substance to be analyzed, to a carrier having a immobilized probe for the substance to be analyzed; and
(B) a step for bonding the immobilized probe on the carrier, to the labeled substance to be analyzed.

The method of detecting a substance to be analyzed of the present invention, uses the quantitative value of the signal intensity for when the slope of an approximated line of the measured signal intensity is within a range between 0.5 and 1.5 times the slope of the approximated line of the previously measured signal intensity, and further comprises: a) a step for supplying a sample solution containing a conjugate A including one substance of a binding pair and a substance to be analyzed, to a porous carrier having a immobilized probe for the substance to be analyzed;
b) a step for reacting a conjugate B including an enzyme and a substance which is specifically bindable to the one substance of the binding pair, with the conjugate A that has been bonded to the immobilized probe on the carrier in the step a), so as to form a conjugate C comprising the conjugate A and the conjugate B;
c) a step for reacting a conjugate D including a substrate for the enzyme in the conjugate B and a detectable labeling substance, with the conjugate C, so as to activate the substrate in the conjugate D, and bonding this activated conjugate D to the porous carrier without performing a pretreatment of loading an external receptor for the substrate; and
d) a step for quantifying the labeling substance in the activated conjugate D on the porous carrier.

By taking the above steps a) to c), firstly the conjugate A including the substance to be analyzed is reacted with the conjugate B which includes the enzyme and is bonded thereto, via the one substance of the binding pair contained in the conjugate A. As a result, a large conjugate C is formed via the probe on the carrier. Next, this conjugate C is reacted with the labeled substrate for the enzyme. The substrate activated by generating the enzyme-substrate reaction, is bonded to the carrier surface. By such an enzyme reaction, the labeling amplified in proportion to the amount of the substance to be analyzed, is formed on the carrier surface, which is then quantified (measured) by the next quantification (measurement) step, so that it becomes possible to provide a more highly sensitive detection method.

Prior to performing the method of detecting a substance to be analyzed of the present invention, probes are immobilized on the carrier. It is not necessary to specifically limit the method of immobilizing, as long as the combination of the probe and the carrier is already known in the technical field. However, it is desirable to employ a method of immobilizing the probes on the carrier in a form where the reaction between the probe and the substance to be analyzed in the step a) is not inhibited. Regarding the probe to be used, one capable of specifically capturing the substance to be analyzed is used. If the substance to be analyzed is a nucleic acid, a nucleic acid molecule having a complementary sequence for at least a part thereof, is used as a probe. If the substance to be analyzed is a protein or a peptide; an antibody capable of specifically recognizing and bonding them, a substrate of an enzyme reaction catalyzed by the protein or the peptide, a cell which is specifically bindable to the protein or the peptide, a phage expressing an antibody for the protein or the peptide on the outer coat, and the like are used as the probe. If the substance to be analyzed is any one in the combination of substances having an already known relation of ligand-receptor, the receptor or the ligand may be used as the probe. In this manner, the substance to be analyzed capable of having a specific relation to bond to the probe includes; a nucleic acid, a sugar, a protein, a peptide, and variously modified substances thereof (such as a phosphorylated protein).

The amount of the substance to be analyzed may be determined based on the common knowledge of a person skilled in the art, considering the amount of probes immobilized on the carrier. On the other hand, if the amount of the substance to be analyzed is extremely small, the amount of probes corresponding to that amount may be immobilized. If the substance to be analyzed is a nucleic acid, it is preferably within a range between 0.01 and 1.0 µg. However, in the present invention, since the detection sensitivity is remarkably improved compared to a conventional case, it may be 0.01 ng or more.

For the carrier used in the present invention, any material can be used as long as it is suitable for the above deposition reaction and the surface area is large. Preferred examples of the carrier include; a carrier substantially capable of holding liquid and capable of moving the liquid in the carrier, and a porous carner. For example, it includes a hollow fiber, a hollow fiber + gel, an aluminum oxide film, an etched silicon wafer, a glass fiber, and a plurality of glass or resin beads held in a container. Examples of the three-dimensional porous carrier include; a gel matrix, a metal oxide film, and a filter made from nylon, nitrocellulose, or a resin. As to the three-dimensional porous carrier of the present invention, preferably an electrochemically produced metal oxide film described in Patent Document 2 (Japanese Patent Publication No. 3208390) is used. As to the method of using the carrier satisfying these preferred conditions, a microarray can be used.

For example, if this microarray is used for detecting the substance to be analyzed, it becomes possible to measure in time series, the signal intensity from a sample such as a nucleic acid which has been detectably (directly) labeled and bonded to an optional probe spot on the array plate, by the hybridization step including the steps (A) and (B). Moreover, the signal intensity for when the slope of an approximated line of the measured signal intensity is within a range between 0.5 and 1.5 times the slope of the approximated line of the previously measured signal intensity, is used. Within this range, it is considered that the hybridization reaction is not saturated. Consequently, by performing measurement in each probe spot within the range, the quantitativeness of each spot is improved. Moreover, the sample such as a nucleic acid bonded to an optional probe spot on the array plate, is bonded with the enzyme via the binding pair, and thereafter the signal intensity from the conversion reaction of the substrate by the enzyme is measured in time series. Furthermore, the signal intensity for when the slope of an approximated line of the measured signal intensity is within a range between 0.5 and 1.5 times the slope of the approximated line of the previously measured signal intensity, is used to calculate the slope of the reaction line in the case where the enzyme reaction in each probe spot is not saturated. By measuring in such a range, the quantitativeness in the case where the substance to be analyzed is indirectly labeled, is improved.

In order to enable detection of one or more substances to be analyzed, on the porous carrier that can be used in the present invention, one or more types of probes for the respective substances to be analyzed are immobilized in respectively separate spots on the porous carrier in a single reaction container. In order to enable measuring of a plurality of samples at the same time, generally the carrier used in the detection method of the present invention has generally a plurality of spots with immobilized probes, and probes for capturing different substances to be analyzed are immobilized in the respective spots. However, the carrier used in the detection method of the present invention may be such that:
- Only one type of probe for one type of substance to be analyzed is immobilized in one spot,
- Only one type of probe for one type of substance to be analyzed is immobilized in a plurality of spots,
- Two types or more probes for one type of substance to be analyzed are immobilized in separate spots by the respective types, or
- Two types or more probes for one type of substance to be analyzed are immobilized in a plurality of spots by the respective types.
The carrier in such a form can be supplied as a carrier exclusively for a specific purpose.

If the porous carrier used in the detection method of the present invention has a two-dimensional probe immobilizable area in the respective spots for immobilizing probes, preferably the size (diameter or diagonal length) of the spots is within a range between 50 and 500 µm. Or, if the probe immobilizable area is three-dimensional, the size (diameter or diagonal length) of the cross-section of the spots in parallel with the base portion of the carrier is within a range between 50 and 500 µm. Furthermore, the porous carrier may have 20 spots or more per one carrier.

In this manner, if the diameter or the diagonal length of the spot (in the case of two-dimensional), or the diameter or the diagonal length of the cross-section thereof (in the case of three-dimensional) is within a range between 50 and 500 µm, about 20 to 400 types of probes can be immobilized in an area having the diameter or the diagonal length of about 4 mm, on the carrier. Furthermore, the volume of the sample to be supplied to the respective spots may be extremely small. Consequently, even in the case where only an extremely small amount of the substance to be analyzed is available, detection using different probes can be simultaneously and effectively performed.

The method of detecting a substance to be analyzed of the present invention also comprises:
a') a step for supplying a sample solution containing a conjugate A including one substance of a binding pair and the substance to be analyzed, to a porous carrier having a immobilized probe for the substance to be analyzed;
b') a step for reacting a conjugate B including an enzyme and a substance which is specifically bindable to said one substance of the binding pair, with the conjugate A that has been bonded to the immobilized probe on the carrier in the step a'), so as to form a conjugate C comprising the conjugate A and the conjugate B;
c') a step for reacting a conjugate D including a substrate for the enzyme in the conjugate B and a detectable labeling substance, with the conjugate C, so as to activate the substrate in the conjugate D, and bonding this activated conjugate D having the activated substrate, to the porous carrier without performing a pretreatment of loading an external receptor for the substrate; and
d') a step for quantifying the labeling substance in the activated conjugate D on the porous carrier.

In the method of detecting a substance to be analyzed of the present invention, since a porous material can be also used for the carrier, in this case it is also possible to drive a solution to the inside and outside of the carrier in each step. That is, the method of detecting a substance to be analyzed of the present invention comprises:
e') a step for supplying a sample solution containing a conjugate A including one substance of a binding pair and the substance to be analyzed, to a porous carrier having a immobilized probe for the substance to be analyzed, and then driving the sample solution to the inside and outside of this porous carrier;
f) a step for supplying a solution containing a conjugate B including an enzyme and a substance which is specifically bindable to said one substance of the binding pair, to the porous carrier, then driving the solution to the inside and outside of this porous carrier, and reacting the conjugate B with the conjugate A that has been bonded to the immobilized probe on the carrier in the step e'), so as to form a conjugate C comprising the conjugate A and the conjugate B;
g') a step for supplying a solution containing a conjugate D including the substrate for the enzyme in the conjugate B and a detectable labeling substance, to the porous carrier, and then impregnating the solution into the porous carrier, reacting the conjugate D with the conjugate C, so as to activate the substrate in the conjugate D, and bonding the activated conjugate D to the porous carrier; and
h') a step for quantifying the labeling substance in the conjugate D on the porous carner.

In this manner, using a porous carrier and appropriately driving the solution to the inside and outside of the carrier has the following advantages. That is, generally, in the case where a large number of substances to be analyzed are to be detected on the same carrier, since there are differences in the optimum condition for detecting the respective substances to be analyzed, it is difficult to detect all substances to be analyzed under optimum conditions. However, in the method of the present invention, a porous carrier is used, and furthermore the sample solution is appropriately driven, the substrate having detectable labeling that is activated by an enzyme is deposed in the vicinity of the area where the substance to be analyzed is captured, and then the signal from the labeling is detected. Moreover, the signal intensity for when the slope of an approximated line of the measured signal intensity is within a range between 0.5 and 1.5 times the slope of the approximated line of the previously measured signal intensity, is used for quantification, so that it becomes possible to perform highly-sensitive measurement while keeping the quantitativeness. Furthermore, it is also possible to suitably perform simultaneous detection of a plurality of substances to be analyzed. Therefore, if the number of spots for immobilizing the probe is 20 or more on the carrier used in the present invention, it becomes possible to highly-sensitively, highly-accurately, and effectively perform simultaneous detection of a plurality of samples.

Here, the one substance of the binding pair is previously bonded to the substance to be analyzed (via an appropriate linker if necessary), so as to form the conjugate (conjugate A). Here, "binding pair" means a combination of substances which are biologically bonded specifically with each other, such as biotin-avidin, biotin-streptavidin, antigen-antibody, and ligand-receptor. The "one substance of the binding pair" means one substance in these combinations. The method of bonding the substance to be analyzed and the one substance of the binding pair can be performed by an already known method/condition in the technical field, and is not specifically limited as long as the form does not inhibit the conjugate formation reaction between the one substance of the binding pair and the other substance of the binding pair in the next step.

In the step a'), by supplying the sample solution containing the conjugate A to the carrier having the immobilized probe, a specific bond between the probe and the substance to be analyzed in the conjugate A is formed. Moreover, since the carrier to be used is porous, if it is appropriately connected to a distributary system comprising a liquid driving device, there may be a step e') for driving the sample solution supplied to the carrier to the inside and outside of the porous carrier. The driving method includes pumping and pipetting of the reaction solution.

The formation of specific bond in the steps a') and e') is performed under a condition which is considered to be optimum for the combination of the substance to be analyzed and the probe. For example, if the substance to be analyzed and the probe are nucleic acids having mutually complementary sequences, the reaction temperature, the composition of the reaction solution, the reaction time, and the like are determined, by considering a Tm value of the complementary double strand formed between the substance to be analyzed and the probe. After this reaction, a step for removing the unreacted substance to be analyzed or the substances not captured by the probe from the reaction system, can be appropriately performed. Normally, the removal step is preferably a washing step using a solution composition with which the resultant product formed by the step is not dissociated. The amount of the washing solution is preferably greater than the amount of the solutions used in the step prior to washing.

In the next step b'), the conjugate B is reacted with the conjugate A. The conjugate B is a substance which is specifically bindable to the one substance of the binding pair, that is, "the other substance of the binding pair" which is previously bonded with an enzyme (via an appropriate linker if necessary). The bonding of the enzyme may be performed by a known method in the technical field, and is not specifically limited as long as the enzyme activity is not inhibited. In this step, similarly to the step e'), it is also possible to drive the solution to the inside and outside of the porous carrier. In this case, it becomes a step f). The driving method includes pumping and pipetting of the reaction solution.

After the reaction of this step b') or step f), the conjugate C is formed on the carrier. This conjugate C is formed such that the binding pair is formed between the conjugate A including (substance to be analyzed-the one substance of the binding pair) and the conjugate B including (the other substance of the binding pair-enzyme), so as to form a larger complex, that is, the conjugate C, as a whole. Moreover, this conjugate C is bonded to the probe immobilized on the carrier, via the substance to be analyzed therein.

After the reaction of the step b') or the step f), a step for removing the unreacted substances that have not been taken into the conjugate C, can be appropriately performed. Normally, the removal step is preferably a washing step using a solution composition with which the resultant product is not dissociated. The amount of washing solution is preferably greater than the amount of solution used in the step prior to washing.

In the next step c') or g'), the conjugate D is reacted with the conjugate C that is formed up to the step b') or f). The conjugate D is made by conjugating the labeling substance with the substrate for the enzyme. Here, the combination of enzyme-substrate is not specifically limited as long as the substrate is activated by the enzyme reaction and the conjugate D including the activated substrate can be bonded onto the carrier surface, and appropriately selected according to the experimental system by a person skilled in the art. Specifically, it includes combinations of an enzyme such as oxidoreductase, hydrolase, lyase, transferase, isomerase, and ligase, with the substrate thereof. The enzyme to be used is preferably horseradish peroxidase (HRP) or alkaline phosphatase.

The concentration of the enzyme to be used (that is, the conjugate B comprising the other substance of the binding pair-enzyme) can be appropriately determined considering the activity of the enzyme and the like, by a person skilled in the art. This concentration varies according to the type of enzyme to be used, and its activity. However, it is generally within a range between 0.8 and 6 pmol/ml. In the present invention, the activated substrate is bonded to, for example, the surface of a carrier made from a metal oxide film, or the surface of a carrier which has been pretreated with a compound including an aromatic amino acid in at least a part thereof.

As to the substrate of the enzyme, substituted phenol such as tyramine, or phosphorylated substituted phenol such as tyrosine phosphate may be used. These substrates are detectably labeled for use. As to the detectable labeling substance of the substrate, a fluorescent substance (such as fluoresceine, alexa, and cyanine), a chemiluminescent substance, a colloidal particle (a metal such as gold and silver, a resin such as latex, a glass, and a ceramics), a fluorescent glass particle, and the like known in the technical field may be used. The selection may be appropriately performed considering the experimental condition and the like, by a person skilled in the art.

If HRP is used for the enzyme, the reaction is necessarily performed under the presence of hydrogen peroxide. In this case, the concentration of hydrogen peroxide is preferably within a range between 0.00008 and 0.0003%, and more preferably between 0.0001 and 0.0002%.

The reaction of using the detection enzyme to activate the substrate so as to depose it on the carrier, is performed at the operative temperature of the detection enzyme to be used, so that the velocity of the deposition reaction can be controlled by temperature. Moreover, it is also possible to firstly directly fluorescent-label the substance to be analyzed with, for example, another labeling substance (distinguishable from the labeling substance bonded to the substrate), and after bonding to the probe, to quantify the substance to be analyzed that has been captured by the probe, by the another labeling substance. In this manner, if the substance to be analyzed is directly labeled, if the quantification result shows that there is an analysis spot having a weak signal intensity which is difficult to analyze, then the conjugate C can be formed by further reacting the conjugate (conjugate B) having the enzyme bonded to a substance which is specifically bindable to the one substance of the binding pair (the other substance of the binding pair) that is bonded with the substance to be analyzed. In this case, next, the substrate of the enzyme, for example, the conjugate (conjugate D) having tyramide and the labeling substance bonded, is reacted with the enzyme, so as to activate the substrate, so that the labeling substance is deposed on the carrier via the activated substrate, to perform detection. As a result, the signal can be amplified, and the signal which had not been detected in the direct labeling method, can be detected. By this method, a large amount of the substance to be analyzed can be detected by the direct labeling method, and a small amount of substance can be detected by the signal amplification method. If only the large amount of the substance to be analyzed is detected, the detection can be performed more easily and quickly. Moreover, by further performing a treatment on the small amount of substance, data of the substance that had been conventionally undetectable, can be obtained.

In the present invention, the steps (1) to (3); (A) to (B); a) to c); a') to c'); or e') to g') are preferably performed within a range between 20°C and 70°C. If the substance to be analyzed is directly labeled (steps (1) to (3), and steps (A) and (B)), this is desirably within a range between 30 to 55°C. If the substance to be analyzed is indirectly labeled (steps a) to c), steps a') to c'), and steps e') to g')), this is desirably within a range between 34 to 40°C (37±3°C). Moreover, these steps are desirably performed at approximately the same temperature within these ranges. For example, if the substance to be analyzed is a nucleic acid and the length of the probe is normally 20 to 80 bases, the hybridization with the probe is preferably performed at about 50 to 65°C, and the enzyme reaction is preferably performed at 20 to 45°C. However, in the present invention, by decreasing the salt concentration of the buffer solution to be used, it is also possible to perform the hybridization and the enzyme reaction at approximately the same temperature. For example, the optimum temperature for hybridization is 50°C when 5 x SSPE is used for the buffer solution. However the optimum temperature for hybridization is 42°C when 1 x SSPE is used for the buffer solution, and it can be within the temperature range at which the enzyme reaction is effectively performed. In this manner, since the hybridization and the enzyme reaction can be performed at approximately the same temperature, the steps (1) to (3); (A) and (B); a) to c); a') to c'); or e') to g') can be performed at approximately the same temperature. Since it becomes unnecessary to change the temperature, the temperature controlling property is improved, thus shortening the measuring time. Moreover, this is particularly preferable since the automation of the reaction device is facilitated. Consequently, more preferably, the steps (1) to (3); (A) and (B); a) to c); a') to c'); or e') to g') are performed at approximately the same temperature.

Moreover, in the next step d') or step h'), by selectively quantifying the signal from the labeling substance in the conjugate D bonded onto the carrier by the above steps, the substance to be analyzed is indirectly quantified. Furthermore, after the step (B) but before the signal intensity measurement; after the step c) but before the signal intensity measurement; after the step c') but before the step d'); or after the step g') but before the step h'); a step for stopping the reaction and/or a step for removing the unreacted substances may be appropriately performed. As to these steps, normally a washing step using a solution composition with which the resultant product bonded to the carrier is not dissociated, is preferred. The amount of the washing solution is preferably greater than the amount of the solutions used in the steps (step (B), step c), step c'), and step g')) prior to washing. The time up to stopping the reaction or starting the washing is preferably set to a predetermined value, considering the existing amount of the substance to be analyzed and the like, based on the results of a preliminary experiment that has been performed in advance.

The step d') or step h') can be performed a plurality of times at optional time intervals after the step c') or step g'). As a result, a substance to be analyzed existing at a low frequency can be also highly-sensitively detected.

As to the combination of the probe/substance to be analyzed, the description is regarding nucleic acids of DNA/DNA, DNA/RNA, or RNA/RNA. However, the combination includes antibody/protein, peptide/protein, protein (ligand)/protein (receptor), sugar/protein, low molecular compound/protein, cell/peptide, cell/protein, and the like. Any one in the above combinations may be either the probe or the substance to be analyzed.

The combination of the one substance of the binding pair bonded to the substance to be analyzed, and a substance which is specifically bindable to the one substance of the binding pair (the other substance of the binding pair) includes hapten/antibody, biotin/avidin or streptavidin, and the like.

The attaching reaction of the substrate by the enzyme in the steps c') and g') can be performed by leaving to stand after the conjugate (conjugate D) including the substrate is impregnated into the carrier by pumping or pipetting. The degree of deposition of the detectable labeling (activated by the enzyme) bonded to the substrate is monitored by a CCD camera, so as to confirm the degree of the reaction, after which the quantification can be performed. Consequently, it is also possible to confirm the degree of the progress of the reaction in each spot. Consequently, data can be measured under optimum conditions while confirming whether the deposition reaction in each spot is in a linearly increasing range or is saturated. Moreover, in order to quantitatively compare data between the respective spots, desirably the brightness of any spot to be compared is the detection limit or more but not saturated. Therefore, when the step d') or h') is performed, desirably, the rate of change in the measured signal intensity is measured, and the substance to be analyzed is quantified using the data for when the slope of an approximated line of the measured signal intensity is within a range between 0.5 and 1.5 times the slope of the approximated line of the previously measured signal intensity The reason is that, if it is within this range, it is possible to consider that a substantial linearity exists between the measurement time and the signal intensity. Most preferred is the case where the slope of the approximated line is approximately constant, that is, the case of a straight line.

The sample solution used in the present invention and/or the washing solution used in the washing step appropriately performed after the respective above steps, may contain a surfactant. In this case, the concentration of the surfactant is preferably within a range between 0.1 and 1%. It is more preferably between 0.3 and 0.7%. The type of the surfactant is selected considering the foamability, the solubility of the reference material into the sample solution, and the like. However, it is preferably selected from cationic surfactants and nonionic surfactants, since the sample solution contains various buffer solutions. It can be selected from those which do not cause precipitation of alkali metal or alkaline-earth metal contained in the sample solution, the washing solution, and the like, nor negatively affect the surface active effect. Specifically, examples thereof include surfactants of fatty acid systems such as sucrose fatty acid ester, sorbitan fatty acid ester, polyoxyethylene sorbitan fatty acid ester, fatty acid alkanolamide, and surfactants of higher alcohol systems such as polyoxyethylene alkylphenyl ether. The most preferred is fatty acid alkanolamide. Among this, lauryl sarcosine [(CH₃(CH₂)₁₀CON(CH₃)(CH₂COOH))] is the most preferred. Moreover, compounds having a similar structure whose length of alkyl group is different, or which have a substituent, are also preferred.

The method of detecting a substance to be analyzed of the present invention, may comprise, the above steps a') to c') or e') to g'), and further comprises after the step c') or g'):
x-1) a step for measuring only a signal intensity from the activated conjugate D in a specific spot on the porous carrier, to confirm in time series whether or not the signal intensity from the specific spot comes to the predetermined signal intensity; and
x-2) a step for, right after confirming that it comes to the predetermined signal intensity in the step x-1), stopping reactions in other spots on the porous carrier, starting to measure the signal intensity from the other spots, and quantifying using the signal intensity for when the slope of an approximated line of the measured signal intensity is within a range between 0.5 and 1.5 times the slope of the approximated line of the previously measured signal intensity.

In this detection method, the specific spot in the step x-1) may be a spot having a immobilized probe for a reference material. Here, the reference material may include a housekeeping gene (such as a β actin gene and a GAPDH gene) that is always expressed in a cell at a constant ratio, and a transcription/translation product thereof (internal reference material).

The degree of the signal from the spot having the reference material captured, that enables the reaction and measurement of the other spots, can be determined by a preliminary experiment. For a greater amount of substances to be analyzed captured by the probe spotted on the carrier, the amount of sample solution, the reaction time, and the like which enable to start/finish the measurement prior to saturation of the signal intensity, can be determined by a preliminary experiment. Consequently, by performing such steps x-1) and x-2) together with the preliminary experiment, data of many spots can be obtained under the condition where the signal intensity is not saturated.

If there are a plurality of specific spots, probes whose immobilized amounts are adjusted in the dilution series at a constant ratio can be immobilized in the plurality of respective specific spots. In addition, probes can be similarly immobilized to the respective spots of all spots including the specific spots or a part of spots other than the specific spots, in a dilution series at a constant ratio. By taking such a structure, even if the quantitation result in one spot shows a signal intensity of less than the detection limit or of the saturation level, since it is possible to use the quantitation result in other spots in the same dilution series, the omission of detecting the specific substance to be analyzed can be prevented. Moreover, if an appropriate preliminary experiment is performed, it can be expected that there is present at least one spot where the linearity is ensured between the probe concentration and the spot signal intensity, in the same dilution series. Consequently, for the dilution series, preferably there are three points of concentration in total which have at least one point each in front and back of one certain concentration. The dilution rate can be appropriately determined by a person skilled in the art, considering the amount of sample to be used, the abundance ratio of the substance to be analyzed, and the like. For example, it may be 2-fold (1/2 times) series, 10-fold (1/10 times) series, and the like.

In the method of the present invention, it may be such that the dilution series of a probe for an external reference material are spotted in the specific spots, and labeled external reference material corresponding to the probe of the external reference material is mixed into the sample solution containing the substance to be analyzed, at an optional concentration, so as to measure the signal intensity from the external reference material. In this case, spots in the dilution series of the probe for the external reference material can be effectively utilized if the amount of the substance to be analyzed is greatly different from the assumption. That is, if the amount of the substance to be analyzed is much greater than the assumption, since the reaction time between the activated substrate and the carrier becomes short, quantitative detection is possible in a spot having a large probe immobilized amount in the dilution series. On the contrary, if the amount of the substance to be analyzed is less than the assumption, since the reaction time between the activated substrate and the carrier becomes long, quantitative detection is possible in a spot having a small immobilized amount.

If detection is performed by measuring the amount of the substance to be analyzed and matching the amount of sample to be added, the external reference material (such as an oligo DNA) is put in always at the same concentration with respect to the detected sample. Then the reaction between the activated substrate and the carrier is stopped so that the external reference material has a predetermined signal intensity, and the data is measured. By so doing, the variance of the reaction between the respective spots can be suppressed. This is effective in a preliminary experiment before the internal reference material is determined, or the like.

If detection is performed without matching the amount of sample to be added for detection, the external reference material (such as an oligo DNA) is put in at the same concentration with respect to the sample to be detected, and then the data is measured in the reaction time allowing the internal reference material to have a predetermined signal intensity. By changing the amount of the sample to be added so as to previously obtain the signal intensity of the external reference material and the data on the concentration of the external reference material, then even if the experiment is performed without actually measuring the amount of the substance to be analyzed, the addition of the substance to be analyzed can be assumed from the signal intensity of the external reference material and the data on the concentration of the external reference material.

In the steps d') and h'), in order to quantify the substrate deposed on the carrier, preferably, a signal (preferably fluorescence intensity) from the labeling of the substrate (preferably fluorescent substance ) is measured using a CCD camera.

In one embodiment, all steps from hybridization to detection can be performed using an FD10 device made by Olympus Corporation.

The present invention can be also applied to various biological substances. For example, a nucleic acid, a sugar, a protein, a peptide, and variously modified substances thereof can be detected. The various modifications of these substances include phosphorylation, methylation, acetylation, and deacetylation. For example, measurement of protein kinase activity is one of these. This protein kinase activity can be performed by measuring whether or not a specific amino acid in a peptide is phosphorylated. In this case, the detection is performed by using a peptide serving as a substrate of the protein kinase, for the probe.

In one example of the present invention, the peptide sequence including a phosphorylation site of a protein serving as the substrate of the protein kinase, is used as a probe, and immobilized on the plate of the porous carrier, and then ATP (ATP-γS) having a sulfur atom artificially introduced in γ-phosphate group is used to perform a phosphorylation reaction. Using the kinase, a sulfur atom of the thiophosphate group introduced into the phosphorylation site of the probe peptide, is reacted with biotin (such as iodoacetyl biotin) having a function group bindable thereto. It is then reacted with a conjugate molecule having the enzyme bonded to avidin which is bindable to biotin. This resultant product is further reacted with another conjugate having a fluorescent substance bonded to the substrate of the enzyme. The substrate activated by the enzyme is immobilized to the phosphorylated probe peptide and the vicinity thereof on the plate, and a fluorescent signal from the immobilized substrate-fluorescent substance is measured. By so doing, it is possible to estimate the amount of the phosphate group introduced into the probe peptide. From this estimate value, it becomes possible to measure the activity of the kinase (substance to be analyzed) supplied for the analysis. Consequently, in the method of the present invention, the activity of an enzyme such as kinase can be easily measured without using radioactive substances, and its application in the field of clinical diagnosis can be also expected in the future.

The method of detecting a substance to be analyzed of the present invention may be not only used in a reaction in a immobilize as described above, but also in a reaction in a liquid-phase. For example, the method may be such that the amount of an enzyme serving as the substance to be analyzed is measured in a glass cell, based on the change in the absorbance depending on the conversion amount of the substrate. Moreover, the method may be such that, a biotin-labeled antibody specific to the substance to be analyzed is bonded to a specific protein serving as the substance to be analyzed that has been immobilized on a membrane, and then the enzyme bonded with streptavidin which is specifically bindable to the biotin, is used, so as to measure the degree of conversion of the substrate due to the enzyme.

The present invention further provides a reagent kit for detecting a substance to be analyzed having in respectively separate containers:
a blocking agent containing a buffer solution containing a monovalent or divalent cation, and at least one type selected from a group consisting of BSA, gelatin, and skim milk;
an enzyme preparation containing a buffer solution containing a monovalent or divalent cation, and at least one type of enzyme selected from a group consisting of oxidoreductase, hydrolase, lyase, transferase, isomerase, and ligase;
a substrate-containing agent containing a conjugate D including a detectable labeling substance and a substrate for the enzyme in the enzyme preparation; and
a washing agent containing a nonionic surfactant and a buffer solution containing a monovalent or divalent cation.

The blocking agent used in the reagent kit for detecting a substance to be analyzed of the present invention contains at least one type of BSA (bovine serum albumin), gelatin, or skim milk, wherein BSA, gelatin, or skim milk is desirably within a range between 0.1 and 10% as a whole. Moreover, this blocking agent may contain 0.1M to 1M NaCI and 3mM to 60mM sodium phosphate. Furthermore, SSPE and the like may be also contained at a desired concentration.

The enzyme preparation used in the reagent kit for detecting a substance to be analyzed of the present invention may contain an enzyme at a concentration desired for the experimental system to be used. However, for example if the enzyme is peroxidase, it is preferably 0.8 to 6 pmol/ml. In the case of an enzyme other than this, the amount thereof within the equivalent range may be added considering its activity and the like. Furthermore, as required, BSA, SSPE, and PBS may be contained at an appropriate concentration.

The substrate-containing agent in the reagent kit for detecting a substance to be analyzed of the present invention may contain a conjugate D including a detectable labeling substance and a substrate for the enzyme in the enzyme preparation, at an appropriate concentration. Moreover, if the enzyme is peroxidase, a substrate-dissolving solution containing hydrogen peroxide with a range between 0.00008% and 0.0003% may be prepared and used.

As described above, the washing agent in the reagent kit for detecting a substance to be analyzed of the present invention contains a surfactant within a range between 0.1 and 1%. In addition to the surfactant, various buffer solution components such as SSPE and PBS may be appropriately contained.

The component concentration of the preparations in the kit is respectively described as the concentration at the time of usage. However, as a kit, it can be a stock solution having several-times the concentration of the final concentration. Moreover, it may be a powder form so that the experimenter can prepare at the time of usage.

### [Example 1]

### (Example 1)

1. The hybridization of nucleic acids was detected using biotin as the one substance of the binding pair bonded with the sample, streptavidin as the substance which is specifically bindable to the one substance of the binding pair, horseradish peroxidase as the enzyme, tyramine as the enzyme substrate, and Alexa488 as the detectable labeling of the enzyme.
2. In the present example, the following materials were used (all were prepared using RNase-Free Water).
   20 x SSPE stock buffer solution (made by Invitrogen) adjusted to pH 6.65
   1% SDS solution (made by Ambion, Inc.)
   PBS/0.01 % tween solution (made by Invitrogen)
   0.0015% hydrogen peroxide solution (made by Molecular Probes Inc.)
   streptavidin-HRP solution (made by Molecular Probes Inc.)
   Tyramide-Alexa488 solution (made by Molecular Probes Inc.)

### (Assay Protocol)

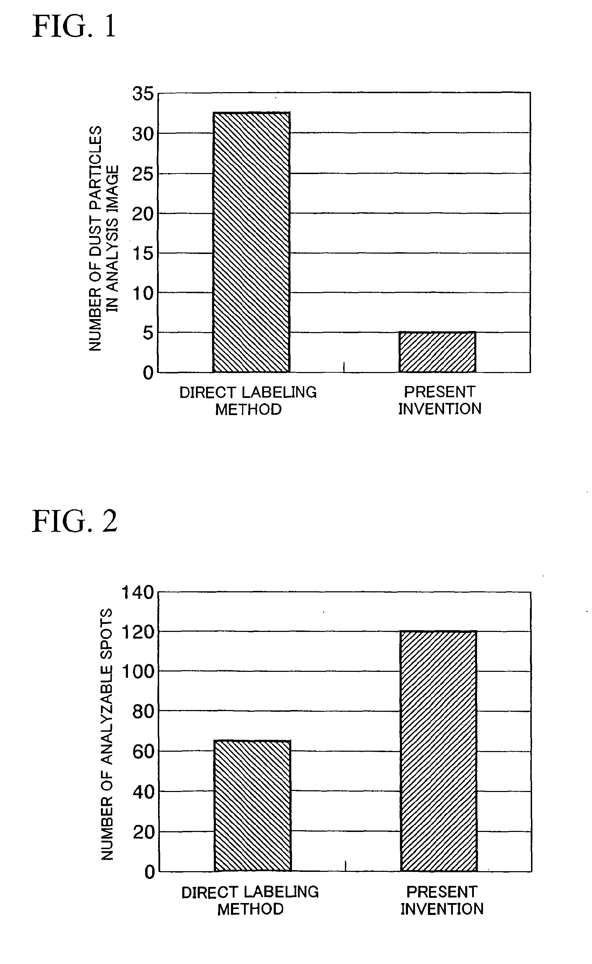

1. The fragmented aRNA sample was prepared as follows. 2 µg of total RNA was extracted from a rat (male whister) liver using NIPPON GENE's ISOGEN (registered trademark), and was used as a template. Using this and Ambion's messageAmp-kit, aRNA was synthesized under the presence of biotinylated UTP. It was fragmented using Ambion's RNA fragmentation-kit in a 1 x fragmentation buffer solution by heating at 70°C for 15 minutes. 5µg of this aRNA was used for hybridization.
2. PamChip (registered trademark) was used as a microarray having immobilized probes. As the probes on this array, 127 probes having the base pair number 60 (SEQ ID NO:1 to 127) were used. Among these sequences, the control sequences are the internal standard gene GAPDH (SEQ ID NO:126) and the external standard gene LAMD (SEQ ID NO:127), which are shown in the sequence listing.
3. An FD10 made by Olympus Corporation was used as an array hybridization and signal detection equipment.
4. The following steps were all performed in the array at 42°C.
5. An array was set in the FD10 device, and 50 µl of PBS-tween was put on the array surface. Then, pumping of the solution was performed for 3 times, so as to prewash the array surface. In this case, pumping means an operation to pass through 50 µl of the solution from the top face of the array to the bottom face of the array, and then to suck to bring it back to the top face of the array in a similar manner.
6. 37.5 µl of aRNA sample aqueous solution was denatured by heating at 99°C for 5 minutes, and then cooling down in ice for 5 minutes. Then, 7.5 µl of 20 x SSPE stock solution was added so as to make 45 µl of 3 x SSPE concentration. The whole amount was put on the prewashed array surface, and then 5 µl of 1% SDS was put thereon.
7. The hybridization of the probes and the sample was performed by pumping 150 times at 42°C.
8. After the hybridization, the solution was removed by a filter paper. 70 µl of 3 x SSPE solution was put thereon, and pumping was performed for 3 times. This was performed for a further 2 times.
9. 20 x SSPE containing 1% BSA was diluted with PBS to make a 3 x SSPE blocking solution. 50 µl of this solution was put on the array surface, and pumping was performed for 5 times.
10. The blocking solution was removed by a filter paper. The streptavidin-horseradish peroxidase conjugate solution which was 100-fold diluted with the blocking solution, was put on the array surface, and pumping was performed for 20 times.
11. The solution was removed by a filter paper. 70 µl of 3 x SSPE was put thereon, and pumping was performed for 3 times. This was repeated for 3 times.
12. 1 µl ofTyramide-Alexa488 was diluted with 100 µl of hydrogen peroxide solution. 50 µl of this solution was supplied to the array surface. Next, 25 µl of the solution was sucked to the bottom face of the array. The lightness of spots on the array surface was visually monitored, with a CCD camera using a GFPHQ filter made by Olympus Corporation, at an exposure time of 50 msec. 10 minutes later, after confirming that the lightness of the lightest most signal spot was saturated, the solution was pushed up to the top face of the array.
13. The solution was removed by a filter paper, and pumping was performed for 3 times using 70 µl of 3 x SSPE.
14. The solution was removed by a filter paper. 30 µl of 3 x SSPE was put thereon, and the whole amount was sucked to the bottom face of the array. The array image was captured with the CCD camera using the GFPHQ filter, at exposure times of 50, 100, 200, and 300 msec. The results are shown in FIG. 1.

### (Comparative Example 1)

As a comparative example, the fragmented aRNA sample directly labeled with a fluorescent substance was prepared as follows. 2 µg of total RNA was extracted from a rat (male wister) liver using NIPPON GENE's ISOGEN, and was used as a template. Using this and Ambion's messageAmp-kit, aRNA was synthesized under the presence of FITC-12-UTP. It was fragmented using Ambion's RNA fragmentation-kit in a 1 x fragmentation buffer solution by heating at 70°C for 15 minutes. 5 µg of this aRNA was used for hybridization. After performing the above reaction steps 2. to 8., the image was captured with a GFPHQ filter at exposure times of 1000 to 5000 msec. The results are shown in FIG 1.

### (Results)

Comparing the case of the signal amplification method of the present invention to the case of the direct labeling using FITC, since the signal intensity was enhanced in the present invention, the exposure time was shortened from 5000 msec to 100 msec. As a result, signals of dust on the array surface that had conventionally been a problem at the time of image analysis, were not observed, and the image analysis was able to be quickly and accurately performed (refer to FIG 1). Moreover, the effect due to "unevenness", that is a problem peculiar to porous arrays, was also reduced. The background value of the image was reduced from 700 to 1000, to 300 to 400, being half or less. The number of analyzable spots was increased by about double, and the low expressive genes became analyzable (refer to FIG. 2).

The time required for the assay was about 2 and a half hours from hybridization to detection, and could be greatly shortened compared to 2 to 3 days in the conventional case where detection is performed using a glass array in a substrate deposition method by means of an enzyme.

### [Example 2]

### (Example 2)

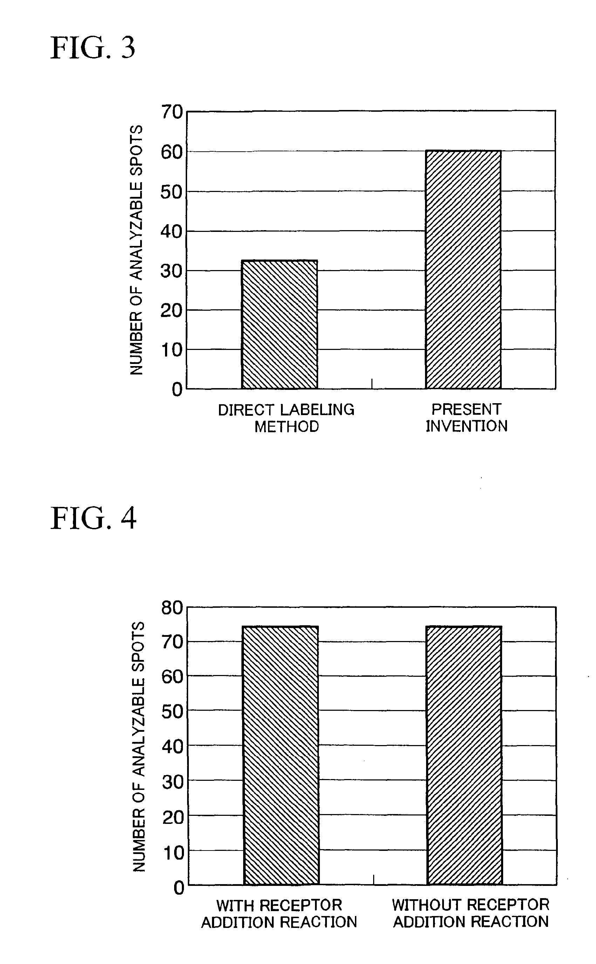

The following experiment was performed in order to confirm whether or not hybridization signals can be detected in the case where only a very small amount of sample to be hybridized is available.
1. The fragmented aRNA sample was prepared as follows. 10 ng of total RNA was extracted from a rat (male wister) liver using NIPPON GENE's ISOGEN (registered trademark), and was used as a template. Using this and Ambion's messageAmp-kit, aRNA was synthesized under the presence of biotinylated UTP. The whole amount was fragmented using Ambion's RNA fragmentation-kit in a 1 x fragmentation buffer solution by heating at 70°C for 15 minutes. The whole amount of this aRNA was used for hybridization.
2. The above reaction steps 2. to 14. in the Example 1 were performed to obtain the data. The results are shown in FIG 3.

### (Comparative Example 2)

1. As a comparative example, the fragmented aRNA sample directly labeled with a fluorescent substance was prepared as follows. 10 ng of total RNA was extracted from a rat (male wister) liver using NIPPON GENE's ISOGEN, and was used as a template. Using this and Ambion's messageAmp-kit, aRNA was synthesized under the presence of FITC-12-UTP. The whole amount was fragmented using Ambion's RNA fragmentation-kit in a 1 x fragmentation buffer solution by heating at 70°C for 15 minutes. The whole amount of this aRNA was used for hybridization. After performing the above reaction steps 2. to 8. in the Example 1, the image was captured with a GFPHQ filter at exposure times of 1000 to 5000 msec. The results are shown in FIG. 3.

### (Results)

As a result of hybridization using the sample aRNA obtained from a very small amount of total RNA, in the case of using the method of the present invention, about a half of the spots in the array were able to be detected. On the other hand, in direct labeling with a fluorescent substance serving as a conventional method, only genes having a high expression level could be detected. By using the present invention, analysis is possible even with a very small amount of sample obtained from a clinical specimen.

Moreover, all the steps had taken 4 to 5 days in the conventional method whereas they could be performed in 2 days in the present example, and thus the time required up to detection could be greatly shortened.

### [Example 3]

In order to confirm whether or not the loading reaction of an external receptor (aromatic amino acid receptor) for loading a tyramide substrate onto the array surface is necessary, the steps of Example 1 were performed in the condition where the step 9. was omitted, and the blocking solution was replaced with a 3 x SSPE solution which was made by diluting 20 x SSPE with PBS in the step 10. The results are shown in FIG 4.

### (Results)

In the case where the loading reaction of the external receptor for the substrate (blocking) was not performed, so as not to load the external receptor for the substrate onto the array surface, the background value of the image was 300 to 400, and the number of analyzable spots was the same as the case where the loading reaction for the external receptor (blocking) was performed. As a result, the present experiment showed that the step for loading the external receptor (aromatic amino acid) for the substrate to the three-dimensional porous carrier, is not necessary (refer to FIG 4).

### [Example 4]

1. The fragmented aRNA sample was prepared as follows. 2 µg of total RNA was extracted from a rat (male whister) liver using NIPPON GENE's ISOGEN, and was used as a template. Using this and Ambion's messageAmp-kit, aRNA was synthesized under the presence of biotinylated UTP. It was fragmented using Ambion's RNA fragmentation-kit in a 1 x fragmentation buffer solution by heating at 70°C for 15 minutes. 5 µg of this aRNA was used for hybridization.
2. As the microarray having immobilized probe nucleic acids, there was used a total of 144 stainless capillary tubes (outer diameter 0.5 mm) filled with an acrylamide gel, that were arranged and adhered 12 lengthwise x 12 widthwise, then cut to a thickness of 5 mm. In the respective capillary tubes, different probe DNAs were mixed in the acrylamide gel and filled therein. The sequences of the probe nucleic acids on the array are described in SEQ ID NO:1 to 127 in the sequence listing.
3. The following steps were all performed in the array set at 42°C.
4. The array was put into a small Tupperware and soaked in PBS-tween, which was then left for 10 minutes. The PBS-tween solution was discarded thereafter.
5. 375 µl of aRNA sample aqueous solution was denatured by heating at 99°C for 5 minutes, and then cooling down in ice for 5 minutes. Next, 75 µl of 20 x SSPE stock solution was added into the denatured sample aqueous solution, so as to make 450 µl of 3 x SSPE concentration. The whole amount was put on the array surface after the above
4. operation, and then 50 µl of 1% SDS aqueous solution was added thereto.
6. The lid of the small Tupperware was closed, and a hybridization reaction was performed overnight under light shaking. After the hybridization, the array was moved into another Tupperware, and added with 5 ml of 3 x SSPE solution. 1 hour of washing by light shaking was performed for three times.
7. 20 x SSPE containing 1% BSA was diluted with PBS to make a 3 x SSPE blocking solution. 1 ml of this solution was put on the array surface, and lightly shaken for 1 hour, to perform a blocking reaction.
8. The BSA blocking solution was discarded. The streptavidin-horseradish peroxidase conjugate containing solution which was 100-fold diluted with the blocking solution, was put on the array surface, and left to stand for 2 hours to perform the reaction.
9. After the reaction, the conjugate containing solution was discarded, and added with 3 x SSPE solution. 1 hour of washing by light shaking was performed for three times.
10. 10 µl of Tyramide-Alexa488 was diluted with 1 ml of 0.0015% hydrogen peroxide solution. The solution was put on the array surface, and left to stand for 10 minutes.
11. The solution containing Tyramide-Alexa488 was discarded, and the array was washed with 3 x SSPE solution for 5 minutes.
12. The array was put on a fluorescence microscope (made by Olympus Corporation) fitted with a CCD camera. The array image was captured using a GFPHQ filter (made by Olympus Corporation), at exposure times of 50, 100, 200, and 300 msec.

### (Comparative Example)

As a control sample, the fragmented aRNA sample directly labeled with a fluorescent substance was prepared as follows.
1. 2 µg of total RNA was extracted from a rat (male wister) liver using NIPPON GENE's ISOGEN (registered trademark), and was used as a template. Using this and Ambion's messageAmp-kit, aRNA was synthesized under the presence ofFITC-12-UTP. It was fragmented using Ambion's fragmentation-kit in a 1 x fragmentation buffer solution by heating at 70°C for 15 minutes. 5 µg of this aRNA was used for hybridization. The above reaction steps 2. to 8. in the Example 4 were performed and, the image was captured with a GFPHQ filter at exposure times of 1000 to 5000 msec.

### (Results)

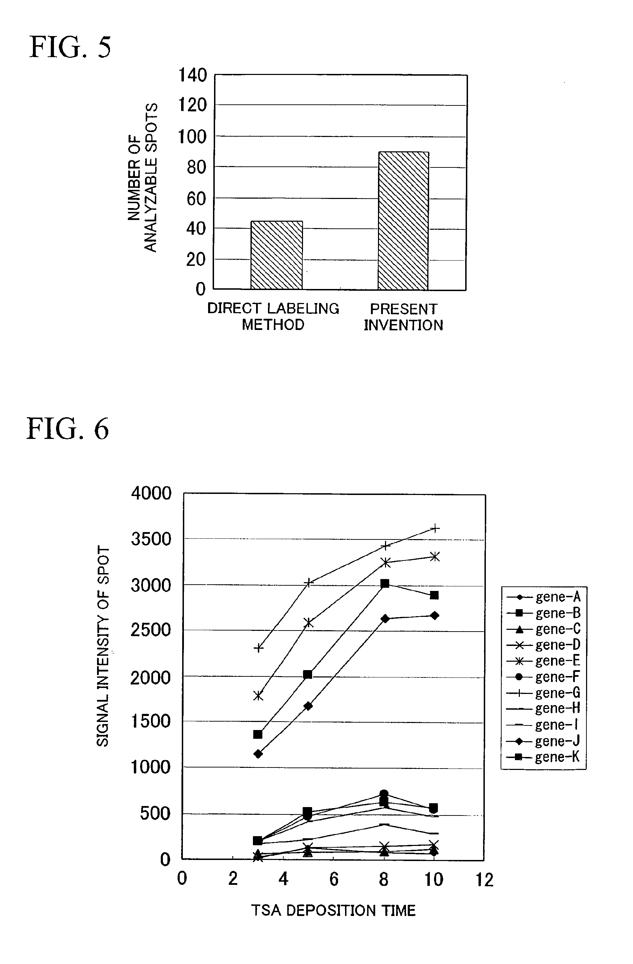

The results of Example 4 and Comparative Example are shown in FIG 5. Comparing the method of the present invention (signal amplification method) to the method of the comparative example (direct labeling), since the signal intensity was enhanced in the method of the present invention, the exposure time was shortened from 5000 msec to 100 msec. The background value of the image was 700 to 1000 in the comparative example, whereas in the method of the present invention this was reduced to 300 to 400 being half or less. The number of analyzable spots was increased by about double in the method of the present invention, and the low expressive gene became analyzable.

### [Example 5]

Two types of samples were used to compare the expression level of the gene to be analyzed.

### A.

Firstly, in order to confirm the time course of the deposition reaction time of tyramide to the porous carrier by means of an enzyme, an experiment comprising the following steps was performed.
1. The fragmented aRNA sample was prepared as follows. 2 µg of total RNA was extracted from a rat (male wister) liver using NIPPON GENE's ISOGEN (registered trademark), and was used as a template. Using this and Ambion's messageAmp-kit, aRNA was synthesized under the presence of biotinylated UTP. 2 µg of this was fragmented using Ambion's RNA fragmentation-kit in a 1 x fragmentation buffer solution by heating at 70°C for 15 minutes. The whole amount of this aRNA was used for hybridization. Next, the reactions from step 2. to 11. in the Example 1 were performed.
12. 1 µl ofTyramide-Alexa488 was diluted with 100 µl of hydrogen peroxide solution. 50 µl of this solution was put on the porous array surface. Next, 25 µl of the solution was sucked to the bottom face of the array, so as to depose tyramide on the carrier for 3, 5, 8, or 10 minutes. Then, the respective solutions were pushed up to the top face of the array, and immediately the following step 13. was performed.
13. The solutions that had been pushed up over the carrier were removed by a filter paper. 70 µl of 3 x SSPE solution was put thereon, and pumping was performed for 3 times.
14. The solution used for the pumping was removed by a filter paper. 30 µl of 3 x SSPE was put thereon, and the whole amount was sucked to the bottom face of the array. The array image was captured with a CCD camera using a GFPHQ filter, at exposure times of 50, 100, 200, and 300 msec.

### (Results)

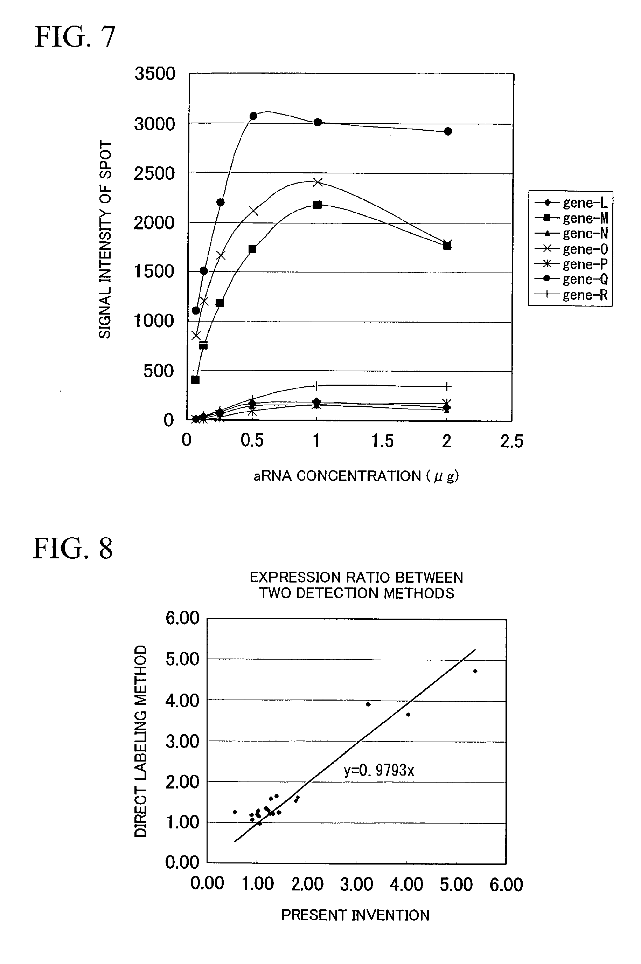

FIG 6 shows the analyzed results of the captured image of the array.

When the time for deposing tyramide on the carrier is changed, in the method of the present invention, the signal spot brightness in the array showed no increase after 8 minutes, and it was found that the tyramide deposition reaction was saturated in about 8 minutes. From this graph, it is further found that both genes having high and low spot signal brightness were changed while the abundance ratio was kept until the signal brightness was saturated. However, once a signal from one of the genes to be analyzed is saturated, the relative ratio with respect to the other genes largely differs thereafter. Consequently, in all genes to be compared on the same array, preferably the substance to be analyzed is detected using a signal intensity before the signal intensity is saturated, that is, when the rate of change in the obtained signal intensity is positive.

The timing of saturation may differ depending on the substance to be analyzed in the sample solution, in some cases. In such case, it is also possible, for example, to use data before all signals of the substance to be analyzed are saturated, and calculate the relative amount of the substance to be analyzed in the sample solution, and then to further continue data acquisition in order to obtain the signal brightness, and then, when the signal intensity of the substance to be analyzed that takes time until saturation, becomes a sufficient value (before the saturation), to use this value to relatively correct the substance to be analyzed which is quickly saturated and the substance to be analyzed which is more slowly saturated, so as to detect all substances to be analyzed.

### B.

Next, in order to calculate the degree of sample amount for showing the spot signal brightness reflecting the aRNA amount of the gene to be analyzed in the sample, the experiment in the method steps 2. to 14. in the Example 5 was performed using 0.065, 0.125, 0.25, 0.5, 1, or 2 µg of the aRNA obtained in step 1. in the Example 5. The tyramide deposition time was 5 minutes.

### (Results)

The results are shown in FIG 7. From this graph, since the signal intensity was not increased and the sample amount and the signal intensity did not correspond to each other even if the amount of aRNA was 1.0 µg or more, then it is found that the proper aRNA amount is in a range between 0.01 and 1.0 µg. Since the data becomes reliable if there is a certain degree of signal intensity, it is preferably 0.05 to 0.75 µg and more preferably 0.1 to 0.5 µg. In the present example, aRNA is used as a sample. However, it is possible to perform the detection under optimum conditions by using the same amount of cDNA as that of the aRNA.

### C.

Next, in order to examine the optimum amount of the horseradish peroxidase (HRP)-streptavidin concentration, the dilution concentration of the substance was set to 4 points of 1/100 (47.2 pmol/ml), 1/1000 (4.72 pmol/ml), 1/4000 (1.18 pmol/ml), and 1/8000 (0.59 pmol/ml). The tyramide deposition time was 5 minutes, and the sample aRNA amount was 0.2 µg, to perform the experiment under the conditions of Example 5 A..

### (Results)

The background value of the array surface tended to decrease as the enzyme concentration was decreased. However, the spot signal brightness was the brightest in the case of 1/4000 dilution. The number of analyzable signal spots and the spot brightness in the cases of 1/100 and 1/8000 dilutions were respectively half or less than the values in the case of 1/4000 dilution. This suggests that an optimum ratio exists in the balance between the enzyme concentration and the substrate concentration since the resultant product of the enzyme and tyramide inhibits the enzyme reaction. Therefore, it is found that the enzyme HRP-streptavidin concentration is appropriately 0.8 to 6 pmol/ml. Since the data becomes reliable if there is a certain degree of signal intensity, the concentration is preferably 0.9 to 4 pmol/ml and more preferably 1 to 2 pmol/ml.

### D.

Taking the experimental results of A to C, the following experiment was performed to detect the difference in the expression level of genes to be analyzed in two types of samples.

The fragmented two types of aRNA samples were prepared as follows. 2 µg of total RNA was extracted from a rat (male wister, phenobarbital administrated group or non-administrated group) liver using NIPPON GENE's ISOGEN, and was used as a template. Using this and Ambion's messageAmp-kit, aRNA was synthesized under the presence of biotinylated UTP. 0.2 µg of each thereof was fragmented using Ambion's RNA fragmentation-kit in a 1 x fragmentation buffer solution by heating at 70°C for 15 minutes. The whole amount of this aRNA was used for hybridization. The reaction steps 2. to 14. in the Example 5 C. having the enzyme amount of 1/4000 were performed to obtain the data.

### (Comparative Example)

As a control, the fragmented aRNA sample directly labeled with a fluorescent substance was prepared as follows. 2 µg of total RNA was extracted from a rat (male wister, phenobarbital administrated group or non-administrated group) liver using NIPPON GENE's ISOGEN, and was used as a template. Using this and Ambion's messageAmp-kit, aRNA was synthesized under the presence of FITC-12-UTP. 2 µg of each thereof was fragmented using Ambion's RNA fragmentation-kit in a 1 x fragmentation buffer solution by heating at 70°C for 15 minutes. The whole amount of this aRNA was used for hybridization. After performing the reactions 2. to 8., the image was captured with a GFPHQ filter at exposure times of 1000 to 5000 msec.

### (Results)

Comparing the expression ratios between the method of the present invention (signal amplification method) and the FITC direct labeling method of the Comparative Example, they were equivalent. However, although the sample amount used in the method of the invention of the present application was very small at 0.2 µg, the number of analyzable spots was equivalent to that in the result of the control (10 times sample amount, 2 µg), and furthermore the expression ratio was accurately maintained (FIG 8).

The tyramide method has been used in the case where the expression of a very small amount of substance has to be highly-sensitively detected, generally such as FISH and CGH. However, if a quantitative experiment is to be performed, the signal is saturated even if the amount is very small, and hence it is not often used for quantitative experiments. In the invention of the present application, it is found that, even in the expression ratio detection method utilizing tyramide, as long as the experiment is performed within optimum ranges of the tyramide deposition time, the sample concentration, and the enzyme amount, then the quantitative experiment can be performed, and the sample amount required therefor may be very small compared to the direct labeling of the conventional method. As a result, since the sample amount required for the analysis may be very small, and furthermore quantitative data can be obtained, the tyramide method can be a very useful method for obtaining data of expression analysis from a microdissection sample or in diagnosis in medical practice where the sample amount is limited, and the like.

### [Example 6]

### A.

In order to detect the kinase phosphorylation reaction using the tyramide method of the invention of the present application, the following experiment was performed. The recombinant cdc2 kinase enzyme activity was measured using the substrate peptide of cdc2 kinase, and an FD10 made by Olympus Corporation was used as the measuring equipment.
1. PamChip used in the Examples 1-5 was used as a three-dimensional porous plate. 8 points in a dilution series (0, 1, 2, 4, 6, 8, 10, and 15 µM) of a peptide sequence Pro-Lys-Thr-Pro-Lys-Lys-Ala-Lys-Leu (made by Promega Corporation) serving as a part of cdc2 kinase substrate (histone H1) were spotted by a spotter, and immobilized on the plate surface by means of covalent bonds.
2. 50 µl of 1% BSA solution in PBS-Tween was added onto the array surface, and the solution was driven to the inside and outside of the plate for 5 times.
3. The recombinant human cdc2 kinase (made by Promega Corporation) was diluted with a solution (25mM MOPs, 10mM MgCl₂, 2mM EDTA, 1mM DTT, 40mM β-glycerophosphate, 20mM p-nitrophenylphosphate, 0.1mM sodium vanadate, and 100 µM ATPγ-S), to make 30 µl, which was then added onto the array surface. The reaction temperature was 37°C, and the sample solution was driven to the top and bottom of the array plate at a pace of once a minute, which was continued for 30 minutes.
4. The reaction solution was pushed up to the top of the array. It was removed by a filter paper. 70 µl of PBS-tween solution was put thereon, and driven for 3 times, so as to remove the unreacted ATP and the enzyme from the array.
5. 150mM Tris-HCI (pH 9.2) and 20 µl of 5mM EDTA were added. Furthermore, 20 µl of 30mM iodoacetyl biotin (made by Pierce Biotechnology, Inc.) solution (50mM phosphate buffer solution, pH 6.0) was added, and then the solution was driven up and down to the inside and outside of the array for 20 times, to perform the reaction. Then, 20 µl of 2% β-ME was added to stop the reaction.
6. The reaction solution was removed by a filter paper. 70 µl of PBS-tween solution was put thereon, and driven for 3 times, so as to wash away the unreacted iodoacetyl biotin.
7. The solution of step 4. was replaced by 50 µl avidin-horseradish peroxidase (made by Vetter Laborbedarf eK) solution that was 4000-folds diluted with PBS-tween, which was then driven up and down for 20 times.
8. The reaction solution was pushed up to the top of the array. It was removed by a filter paper. 70 µl of PBS-tween solution was put thereon, and driven for 3 times, so as to wash away the unreacted enzyme.
9. 50 µl of Tyramide-Alexa488 solution (made by Molecular Probe Inc.) that was 100-folds diluted with 0.0015% H₂O₂ solution was put on the array surface, 25 µl of which was sucked downward and left to stand for 5 minutes. Then, the solution was pushed up to the array surface. It was removed by a filter paper. 70 µl of PBS-tween solution was put thereon, and driven up and down once.
10. The PBS-tween solution was replaced by 30 µl of PBS-tween solution. Then, the image was captured using a GFPHQ filter, at exposure times of 50 to 800 msec.

### (Results)

The spot signal brightness was increased in proportion to the concentration gradient of the substrate peptide spotted on the array.

### B.

Next, in order to evaluate whether or not the phosphorylation reaction of the above A. is a specific reaction, the inhibition experiment of cdc2 kinase was performed using Olomoucine (made by Promega Corporation) serving as a specific inhibitor of cdc2 kinase.

0, 25, 50, or 100 µM of Olomoucine was added into the reaction solution in the step 3. in the Example 5 A., and the same experiment as that of the Example 5 A. was performed.

### (Results)

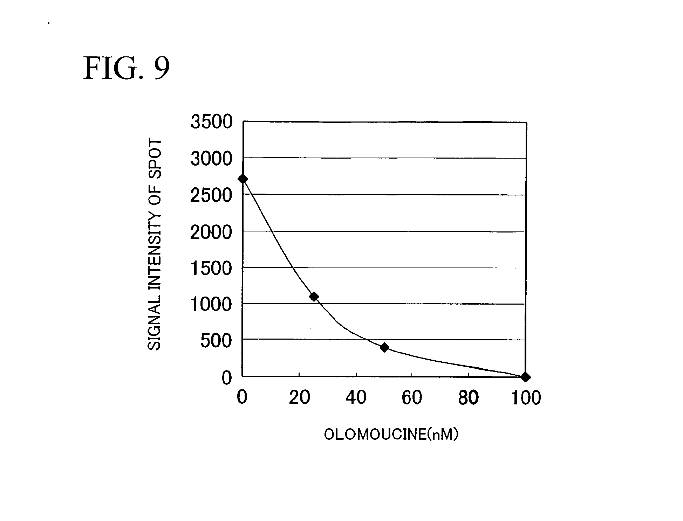

The results are shown in FIG 9. The signal brightness of the peptide spot was decreased depending on the Olomoucine concentration, and it was found that the phosphorylation reaction of cdc2 kinase was inhibited. As a result, it was found that this reaction is a specific reaction. Furthermore, by the experiments of A and B, it was found that it is possible to immobilize the kinase substrate peptide on the porous plate surface, so as to measure the kinase activity by the tyramide method.

### [Example 7]

1. Aspiration biopsy was performed from a rat (male wister, phenobarbital administrated group or non-administrated group) liver, and total RNA was extracted from the whole amount of the obtained tissues (about 5 mg) using NIPPON GENE's ISOGEN. Using the whole obtained amount and Ambion's messageAmp-kit, aRNA was synthesized under the presence of biotinylated UTP.
2. Next, the hybridization was performed using the whole amount of the aRNA synthesized in 1. The array used for the hybridization had a probe (SEQ ID NO:127) of the external standard gene LAMD spotted in a dilution series of 100, 10, 1, 0.1, 0.01, 0 µM concentration as an oligo concentration at the time of spotting, in addition to the probes of the gene group used for Example 1. Moreover, the sample at the time of hybridization was mixed with the same concentration (10pM) of oligo DNA having a complementary sequence with this sequence.
3. The steps 1 to 12. of Example 1 were performed under the optimum conditions obtained in Example 5 and at 0.00015% of hydrogen peroxide concentration. Then the tyramide solution was put on the array surface, a half of which was sucked into the array surface. The image of the array surface was continuously captured at an exposure time of 50 msec.
4. The spot signal intensity of the internal standard gene GAPDH was monitored. When it came to 2500, the whole amount of the tyramide solution was pushed up to the array surface having the respective samples thereon, so as to exchange the washing liquid, and washing was performed thereafter. The final image capturing was performed at exposure times of 100, 500, 1000, and 2000 msec.
5. As a control experiment, the experiment was performed on all samples having the step of 4. for a fixed time of 5 minutes.
6. In the experiment (Table 1) where the tyramide reaction time was adjusted by the spot signal intensity of the internal standard gene GAPDH in the 1. to 4., the signal intensity of the internal standard gene in the image captured by the final image capturing was the same among both samples of the administrated group and the non-administrated group, and the results showing almost the same expression ratio as that of the experiment in the case where the addition sample amount was quantified and added for the same amount in Example 5, could be obtained (Table 2). On the other hand, in the control group, the tyramide reaction was saturated in many spots of the non-administrated group for the same tyramide reaction time, and even if it was corrected using the signal intensity of the internal standard gene, the expression ratio largely differed from that of Example 5 (Table 3).

**Table 1**

| | Control | Phenobal | |
|---|---|---|---|
| aRNA | 100ng | 400ng | |
| Deposition Time | 7 min. | 2 min. | |
| Gene | Raw Data | Raw Data | Ratio (Phenobal/Control) |
| A | 380 | 680 | 1.8 |
| B | 270 | 750 | 2.8 |
| C | 500 | 250 | 0.5 |
| D | 1500 | 2800 | 1.9 |
| E | 3000 | 500 | 0.2 |
| F | 50 | 60 | 1.2 |
| GAPDH | 2500 | 2500 | 1.0 |

**Table 2**

| | Control | Phenobal | |
|---|---|---|---|
| aRNA | 200ng | 200ng | |
| Deposition Time | 5 min. | 5 min. | |
| Gene | Raw Data | Raw Data | Ratio (Phenobal/Control) |
| a | 370 | 650 | 1.8 |
| b | 290 | 745 | 2.6 |
| c | 510 | 255 | 0.5 |
| d | 1350 | 2900 | 2.1 |
| e | 3000 | 550 | 0.2 |
| f | 60 | 65 | 1.1 |
| GAPDH | 2500 | 2500 | 1.0 |

**Table 3**

| | Control | Phenobal | |
|---|---|---|---|
| aRNA | 100ng | 400ng | |
| Deposition Time | 5 min. | 5 min. | |
| Gene | Raw Data | Raw Data | Ratio (Phenobal/Control) |
| a | 270 | 1700 | 2.6 |
| b | 190 | 1875 | 4.1 |
| c | 350 | 625 | 0.7 |
| d | 1100 | 4095 | 1.6 |
| e | 2150 | 1250 | 0.2 |
| f | 40 | 150 | 1.6 |
| GAPDH | 1700 | 4095 | 1.0 |

7. Next, the signal in a dilution series of the external standard gene was saturated at the concentration of 10 µM in the administrated group, and no saturation was seen even at a concentration of 100 µM in the non-administrated group. In the preliminary experiment, there was a result of hybridization where the oligo DNA of LAMD gene serving as the external standard gene was set constant and the dilution series of the sample concentration were made. Comparing with this result, it could be estimated that the sample concentration used for hybridization was about aRNA 100 ng in the administrated group and about aRNA 400 ng in the non-administrated group. In the control experiment, since the tyramide reaction time and the exposure time for image capturing were the same, the signal intensity of LAMD was the same among both samples.
8. From the present experiment, it is found that in the present method, even in the hybridization of an unknown sample amount, by stopping the reaction when the spot signal intensity of the internal standard gene is the same at the time of the tyramide reaction, to obtain the signal, it is possible to obtain data of an accurate expression ratio, and by putting the fixed amount of the external standard thereinto, it is possible to accurately estimate the sample amount which was firstly put in.
9. Moreover, in the present method, if the signal intensity of the internal standard gene GAPDH is saturated right after the tyramide reaction solution was added, since the amount of the sample nucleic acid to be put in can be estimated to be a quantitative limit or more (1 µg or more), it is possible to dilute the sample so as to stimulate it to perform the experiment again.
10. The present method is particularly effective if the sample to be put in is 1 µg or less. In the case where only a very small amount of sample is available such as in fine needle biopsy, the detection is possible by means of signal amplification, and accurate measurement can be performed.

### [Example 8]

### (Effect of concentration of hydrogen peroxide solution)

The experiment was performed to determine whether or not the signal intensity is affected by the concentration of hydrogen peroxide serving as the substrate at the time of the tyramide reaction.
1. The steps 1. to 14. in the method of Example 4 were performed, then hydrogen peroxide solution which dissolves tyramide-Alexa488 was added within a concentration range between 0.000015 and 0.0015 (%).
2. As a result, when the 0.00015% hydrogen peroxide solution was used, the array signal intensity was 1.5 times brighter and the background value was lower, compared to those in the case of 0.000015% or 0.0015%.
3. From the comparison results of the signal intensity and the background intensity, the hydrogen peroxide solution concentration at the time of the tyramide reaction was preferably 0.00008 to 0.0003%, and more preferably 0.0001 % to 0.0002%.

### [Example 9]

### (Effect of adding surfactant)

An examination was made to determine whether or not the signal is affected by adding a surfactant at the time of hybridization or at the time of washing.
1. In the steps 1. to 14. in the method of Example 4, the hybridization buffer solution or the washing liquid was replaced with 3 x SSPE containing lauryl sarcosine within a range between 0.001 % and 5%.
2. When 3 x SSPE containing 0.5% lauryl sarcosine was used, the signal intensity was increased to double and the background value was decreased to about a half, compared to the case where it was not contained.
3. From the comparison result of the signal intensity and the background intensity, the lauryl sarcosine concentration at the time of the tyramide reaction was preferably 0.1 to 1%, and more preferably 0.3% to 0.7%.

### [Example 10]

1. An aspiration biopsy was performed from a rat (male wister, phenobarbital administrated group or non-administrated group) liver, and total RNA was extracted from the whole amount of the obtained tissues (about 5 mg) using NIPPON GENE's ISOGEN (registered trademark). 0.5 µg of this and QIAGEN's Label-star was used, and cDNA was synthesized under the presence of biotinylated UTP.
2. Next, the hybridization was performed using 0.1 ng of the cDNA synthesized in 1.
3. The steps of 1. to 12. of Example 1 were performed under the optimum condition obtained in Example 5 and at 0.00015% of hydrogen peroxide concentration. Then the tyramide solution was put on the array surface, a half of which was sucked from the array surface to the inside. The image of the array surface was continuously captured at an exposure time of 50 msec. When GAPDH came to the value of 2500, the reaction was stopped and data was obtained.
4. The result of hybridization was equivalent to that of Example 5 (result is not shown). If cDNA is used, since the fragment length of cDNA is longer than that of aRNA, it is considered to be possible to detect the signal with a less amount than the aRNA amount. As a result, if the substance to be analyzed is a nucleic acid, the appropriate amount is 0.01 ng to 1 µg.

### [Example 11]

### (Example 11)

1. An aspiration biopsy was performed from a rat (male wister, phenobarbital administrated group or non-administrated group) liver, and total RNA was extracted from the whole amount of the obtained tissues (about 5 mg) using NIPPON GENE's ISOGEN (registered trademark). Using the whole obtained amount and Ambion's messageAmp-kit, aRNA was synthesized under the presence ofFITC-12-UTP.
2. Next, the hybridization was performed using the whole amount of the aRNA synthesized in 1. A slide glass was used as the microarray plate. As the probes on this array, 127 probes having the base pair number 60 (SEQ ID NO:1 to 127) were used. Among these sequences, the control sequences are the internal standard gene GAPDH (SEQ ID N0:126) and the external standard gene LAMD (SEQ ID N0:127), which are shown in the sequence listing.
3. 375 µl of aRNA sample aqueous solution was denatured by heating at 99°C for 5 minutes, and then cooling down in ice for 5 minutes. Next, 75 µl of 20 x SSPE stock solution was added to the denatured sample aqueous solution, so as to make 450 µl of 3 x SSPE concentration. Then, the whole amount was put on the slide glass array surface of 2.
4. The hybridization reaction was performed while the slide glass array was lightly shaken. The image of the array surface was captured with a CCD camera each hour. When the signal intensity from the spot of the internal standard gene GAPDH came to 2500, each array was moved into another Tupperware, and added with 5 ml of 3 x SSPE solution. 1 hour of washing by light shaking was performed for three times.
5. The array was air-dried, then the fluorescence intensity was measured with a CCD camera.

### (Comparative Example)

An experiment of the same structure as that of Example 11 was performed to measure the signal intensity, except that the hybridization was performed overnight to capture the array image without quantifying the signal intensity derived from the internal standard gene, instead of monitoring the progress of the hybridization reaction with a CCD camera each hour, and previously stopping the reaction when the signal intensity derived from the internal standard gene came to a constant level.

### (Result)

In the Example 11, the signal intensity derived from the internal standard gene in the image captured in the final image capturing in time series was at the same level among both samples of the phenobarbital administrated group and the non-administrated group, and results showing almost the same expression ratio as that of the experiment in Example 5 (in the case where the addition sample amount was quantified and added for the same amount), could be obtained. On the other hand, in the Comparative Example, many spots of the non-administrated group were saturated for the same hybridization reaction time, and even if it was corrected using the signal intensity derived from the internal standard gene, the expression ratio largely differed from that of the experiment performed by adjusting the sample amount.

### [Example 12]

The experiment of the same structure as that of Example 11 was performed, except that a carrier having a porous aluminum anode oxide film was used instead of using the slide glass array of Example 11, and furthermore the signal intensity was measured while the sample solution was moved to the inside and outside of the carrier by a pump.

### (Result)

Comparing with the case of the Example 11, a similar result to that of the Example 11 could be obtained for a time length of about 1/20.

### INDUSTRIAL APPLICABILITY

The method of detecting a substance to be analyzed of the present invention can be used in the examination industry, the medical industry, and the like, as an examination method for diagnosing a disease.

## Claims

1. A method of detecting a substance to be analyzed, comprising:
(1) a step for measuring a intensity of a signal derived from a labeled substance to be analyzed, in a time series during an increase in the signal intensity;
(2) a step for comparing a specific signal intensity to a predetermined value; and
(3) a step for quantifying the substance to be analyzed, when the specific signal intensity comes to the predetermined value, using the signal intensity having that value.

2. A method of detecting a substance to be analyzed, comprising the steps of:
measuring a intensity of a signal derived from a labeled substance to be analyzed, in a time series during an increase in the signal intensity; and
representing the signal intensity by a function of time, and using the quantitative value of the signal intensity for when the slope of an approximated line of the measured signal intensity is within a range between 0.5 and 1.5 times the slope of the approximated line of a previously measured signal intensity.

3. A method of detecting a substance to be analyzed according to claim 2, comprising, prior to the step of measuring the signal intensity:
(A) a step for supplying a sample solution containing the labeled substance to be analyzed, to a carrier having a immobilized probe for the substance to be analyzed; and
(B) a step for bonding the immobilized probe on the carrier, to the labeled substance to be analyzed.

4. A method of detecting a substance to be analyzed according to claim 2, comprising, prior to the step of measuring the signal intensity:
a) a step for supplying a sample solution containing a conjugate A including one substance of a binding pair and the substance to be analyzed, to a carrier having a immobilized probe for the substance to be analyzed;
b) a step for reacting a conjugate B including an enzyme and a substance which is specifically bindable to said one substance of the binding pair, with the conjugate A that has been bonded to the immobilized probe on the carrier in the step a), so as to form a conjugate C comprising the conjugate A and the conjugate B; and
c) a step for reacting a conjugate D including a substrate for the enzyme in the conjugate B and a detectable labeling substance, with the conjugate C, so as to activate the substrate in the conjugate D, and bonding this activated conjugate D having the activated substrate, to the carrier without performing a pretreatment of attaching an external receptor for the substrate.

5. A method of detecting a substance to be analyzed, comprising:
a') a step for supplying a sample solution containing a conjugate A including one substance of a binding pair and a substance to be analyzed, to a porous carrier having a immobilized probe for the substance to be analyzed;
b') a step for reacting a conjugate B including an enzyme and a substance which is specifically bindable to said one substance of the binding pair, with the conjugate A that has been bonded to the immobilized probe on the carrier in the step a'), so as to form a conjugate C comprising the conjugate A and the conjugate B;
c') a step for reacting a conjugate D including a substrate for the enzyme in the conjugate B and a detectable labeling substance, with the conjugate C, so as to activate the substrate in the conjugate D, and bonding this activated conjugate D having the activated substrate, to the porous carrier without performing a pretreatment of attaching an external receptor for the substrate; and
d') a step for quantifying the labeling substance in the activated conjugate D on the porous carrier.

6. A method of detecting a substance to be analyzed, comprising:
e') a step for supplying a sample solution containing a conjugate A including one substance of a binding pair and a substance to be analyzed, to a porous carrier having a immobilized probe for the substance to be analyzed, and then driving the sample solution to the inside and outside of this porous carrier;
f) a step for supplying a solution including a conjugate B including an enzyme and a substance which is specifically bindable to said one substance of the binding pair, to the porous carrier, then driving the solution to the inside and outside of this porous carrier, and reacting the conjugate B with the conjugate A that has been bonded to the immobilized probe on the carrier in the step e'), so as to form a conjugate C comprising the conjugate A and the conjugate B;
g') a step for supplying a solution containing a conjugate D including the substrate for the enzyme in the conjugate B and a detectable labeling substance, to the porous carrier, and then impregnating the solution into the porous carrier, reacting the conjugate D with the conjugate C, so as to activate the substrate in the conjugate D, and bonding the activated conjugate D to the porous carrier; and
h') a step for quantifying the labeling substance in the conjugate D on the porous carrier.

7. A method of detecting a substance to be analyzed, according to any one of claim 1 through claim 6, wherein the steps (1) to (3); (A) to (B); a) to c); a') to c'); or e') to g') are performed within a range between 20°C to 70°C.

8. A method of detecting a substance to be analyzed, according to any one of claim 1 through claim 6, wherein the steps (1) to (3); (A) to (B); a) to c); a') to c'); or e') to g') are performed within a range between 20°C and 70°C, at approximately the same temperature.

9. A method of detecting a substance to be analyzed, according to any one of claim 3 through claim 6, comprising a step for washing the carrier: after the step (B) but before the signal intensity measurement; after the step c) but before the signal intensity measurement; after the step c') but before the step d'); or after the step g') but before the step h').

10. A method of detecting a substance to be analyzed, according to claim 5, wherein the step d') is performed a plurality of times at optional time intervals after the step c').

11. A method of detecting a substance to be analyzed, according to claim 6, wherein the step h') is performed a plurality of times at optional time intervals after the step g').

12. A method of detecting a substance to be analyzed, according to either one of claim 10 and claim 11, wherein: in the step d') or h'), the measured signal intensity is represented by a function of time, and the quantification is performed using the signal intensity for when the slope of an approximated line of the measured signal intensity is within a range between 0.5 and 1.5 times the slope of the approximated line of the formerly measured signal intensity.

13. A method of detecting a substance to be analyzed, comprising the following steps a') to c') or e') to g'):
a') a step for supplying a sample solution containing a conjugate A including one substance of a binding pair and a substance to be analyzed, to a porous carrier having a immobilized probe for the substance to be analyzed;
b') a step for reacting a conjugate B including an enzyme and a substance which is specifically bindable to said one substance of the binding pair, with the conjugate A that has been bonded to the immobilized probe on the carrier in the step a'), so as to form a conjugate C comprising the conjugate A and the conjugate B;
c') a step for reacting a conjugate D including a substrate for the enzyme in the conjugate B and a detectable labeling substance, with the conjugate C, so as to activate the substrate in the conjugate D, and bonding this activated conjugate D having the activated substrate, to the porous carrier without performing a pretreatment of attaching an external receptor for the substrate;
e') a step for supplying a sample solution containing a conjugate A including one substance of a binding pair and the substance to be analyzed, to a porous carrier having a immobilized probe for the substance to be analyzed, and then driving the sample solution to the inside and outside of this porous carrier;
f) a step for supplying a solution containing a conjugate B including an enzyme and a substance which is specifically bindable to said one substance of the binding pair, to the porous carrier, then driving the solution to the inside and outside of this porous carrier, and reacting the conjugate B with the conjugate A that has been bonded to the immobilized probe on the carrier in the step e'), so as to form a conjugate C comprising the conjugate A and the conjugate B; and
g') a step for supplying a solution containing a conjugate D including the substrate for the enzyme in the conjugate B and a detectable labeling substance, to the porous carrier, and then impregnating the solution into the porous carrier, reacting the conjugate D with the conjugate C, so as to activate the substrate in the conjugate D, and bonding the activated conjugate D to the porous carrier; and
further comprising, after the step c') or g'):
x-1) a step for measuring only a signal intensity from the activated conjugate D in a specific spot on the porous carrier, to confirm in time series whether or not the signal intensity from the specific spot comes to the predetermined signal intensity; and
x-2) a step for, right after confirming that it comes to the predetermined signal intensity in the step x-1), stopping reactions in other spots on the porous carrier, starting to measure the signal intensity from the other spots, representing the signal intensity by a function of time, and measuring the amount using the signal intensity for when the slope of an approximated line of the measured signal intensity is within a range between 0.5 and 1.5 times the slope of the approximated line of the formerly measured signal intensity.

14. A method of detecting a substance to be analyzed, according to claim 13, wherein the specific spot in the step x-1) is a spot having a immobilized probe for a reference material.

15. A method of detecting a substance to be analyzed, according to claim 14, wherein there are plurality of the specific spots, and probes whose immobilized amounts are adjusted in a dilution series at a constant ratio are immobilized in the respective spots.

16. A method of detecting a substance to be analyzed, according to claim 9, wherein the amount of washing liquid used in the washing step is greater than the amount of solution used in the step (B), c), c'), or g').

17. A method of detecting a substance to be analyzed, according to any one claim 1, claim 2, claim 5, claim 6, and claim 13, wherein the substance to be analyzed is a nucleic acid, a sugar, a protein, a peptide, or a modified substance thereof.

18. A method of detecting a substance to be analyzed, according to claim 17, wherein, if the substance to be analyzed is a nucleic acid, the amount of the substance to be analyzed is within a range between 0.01 ng and 1.0 µg.

19. A method of detecting a substance to be analyzed, according to any one of claim 4, claim 5, claim 6, and claim 13, wherein the enzyme is at least one type of enzyme selected from a group consisting of an oxidoreductase, a hydrolase, a lyase, a transferase, an isomerase, and a ligase.

20. A method of detecting a substance to be analyzed, according to claim 19, wherein the amount of the enzyme is within a range between 0.8 pmol/ml and 6 pmol/ml.

21. A method of detecting a substance to be analyzed, according to any one of claim 4, claim 5, claim 6, and claim 13, wherein the binding pair is selected from a group consisting of biotin-avidin, biotin-streptavidin, antigen-antibody, and ligand-receptor.

22. A method of detecting a substance to be analyzed, according to any one of claim 4, claim 5, claim 6, and claim 13, wherein the substrate is a substituted phenol or a phosphorylated substituted phenol.

23. A method of detecting a substance to be analyzed, according to any one of claim 4, claim 5, claim 6, and claim 13, wherein the enzyme is a peroxidase, and the step c), c'), or g') is performed under the presence of hydrogen peroxide having a concentration of 0.00008 to 0.0003%.

24. A method of detecting a substance to be analyzed, according to any one of claim 1, claim 2, claim 5, claim 6, and claim 13, wherein the sample solution includes a surfactant.

25. A method of detecting a substance to be analyzed, according to claim 9, wherein the washing solution used in the washing step includes a surfactant.

26. A method of detecting a substance to be analyzed, according to any one of claim 5, claim 6, and claim 13, wherein the material of the porous carrier is a metal oxide film.

27. A method of detecting a substance to be analyzed, according to any one of claim 5, claim 6, and claim 13, wherein the porous carrier is pretreated with a compound containing an aromatic amino acid in at least a part thereof.

28. A method of detecting a substance to be analyzed, according to any one of claim 1, claim 2, claim 5, claim 6, and claim 13, wherein there are one or more types of the substances to be analyzed, and one or more types of probes for the respective substances to be analyzed are immobilized in respectively separate spots on a porous carrier in a single reaction container.

29. A method of detecting a substance to be analyzed, according to either one of claim 10 and claim 11, wherein there are two or more of the substances to be analyzed, the method further comprising a step for comparing the quantitative values of the two or more substances to be analyzed, using the signal intensity for when the signal intensity from the labeling substance is a predetermined value.

30. A method of detecting a substance to be analyzed, according to any one of claim 1, claim 2, claim 5, claim 6, and claim 13, wherein: the diameter or the diagonal length of the spots if the probe immobilizable area in the spots is two-dimensional, or the diameter or the diagonal length of the cross-section of the spots in parallel with the base portion of the carrier if the probe immobilizable area in the spots is three-dimensional, is within a range between 50 and 500 µm; and the number of the spots on a single carrier is 20 or more.

31. A reagent kit for detecting a substance to be analyzed having in respectively separate containers:
a blocking agent containing a buffer solution containing a monovalent or divalent cation, and at least one type selected from a group consisting of BSA, gelatin, and skim milk;
an enzyme preparation containing a buffer solution containing a monovalent or divalent cation, and at least one type of enzyme selected from a group consisting of oxidoreductase, hydrolase, lyase, transferase, isomerase, and ligase;
a substrate-containing agent containing a conjugate D including a detectable labeling substance and a substrate for the enzyme in the enzyme preparation; and
a washing agent containing a nonionic surfactant and a buffer solution containing a monovalent or divalent cation.

32. A reagent kit for detecting a substance to be analyzed having in respectively separate containers:
a blocking agent containing 0.1M to 1M NaCl, 3mM to 60mM sodium phosphate, and 0.1% to 10% of at least one type selected from a group consisting of BSA, gelatin, and skim milk;
an enzyme preparation containing 0.8 pmol/ml to 6 pmol/ml peroxidase, and a buffer solution containing a monovalent or divalent cation;
a substrate-containing agent containing a conjugate D including a detectable labeling substance and a substrate for the enzyme in the enzyme preparation;
a substrate dissolving solution containing 0.00008% to 0.0003% hydrogen peroxide; and
a washing liquid containing 0.1 % to 1% nonionic surfactant, 0.1M to 1M NaCl, and 3mM to 60mM sodium phosphate.
